# EUROPEAN PATENT SPECIFICATION

(11) **EP 3 556 369 B1**
(45) Date of publication and mention of the grant of the patent: **14.04.2021**
(21) Application number: 17880064.5
(22) Date of filing: 15.12.2017
(51) Int. Cl.: A61K 31/502, A61K 9/22, A61K 9/52, A61P 35/00, A61K 9/16, A61K 9/20, A61K 9/24, A61K 9/50

(54) **OLAPARIB ORAL SUSTAINED AND CONTROLLED RELEASE PHARMACEUTICAL COMPOSITION AND USES THEREOF**
ORALE PHARMAZEUTISCHE ZUSAMMENSETZUNG MIT VERZÖGERTER UND KONTROLLIERTER FREISETZUNG VON OLAPARIB UND DEREN VERWENDUNGEN
COMPOSITION PHARMACEUTIQUE ORALE À LIBÉRATION PROLONGÉE ET CONTRÔLÉE D'OLAPARIB ET SES UTILISATIONS

(30) Priority: 16.12.2016 CN 201611168797
(43) Date of publication of application: 23.10.2019
(73) Proprietor: Shanghai Institute of Materia Medica, Chinese Academy of Sciences, Shanghai 201203 (CN)
(72) Inventor: GAN, Yong, Shanghai 201203 (CN); ZHU, Quanlei, Shanghai 201203 (CN); GUO, Shiyan, Shanghai 201203 (CN); ZHU, Chunliu, Shanghai 201203 (CN)
(74) Representative: Grünecker Patent- und Rechtsanwälte PartG mbB
(86) International application number: PCT/CN2017/116475
(87) International publication number: WO 2018/108152

(56) References cited:
- WO-A1-2015/031536
- WO-A1-2015/031536
- CN-A- 102 238 945
- CN-A- 103 417 505
- CN-A- 104 873 457
- US-A1- 2016 008 473

## Description

### Technical Field

The invention relates to the field of olaparib pharmaceutical preparations, in particular to an oral sustained and controlled release pharmaceutical composition of olaparib and the use thereof.

### Background Art

Olaparib, whose chemical name is 1-(cyclopropylcarboxyl)-4-[5-[(3,4-dihydro-4-oxo-1-phthalazinyl)methyl]-2-fluorobenzoyl]piperazine with a molecular formula of C₂₄H₂₃FN₄O₃ and a molecular weight of 434.46, has the following chemical structure:

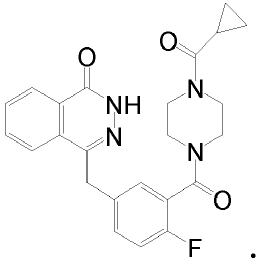

Olaparib (trade name: LYNPARZAR) is a new drug developed by AstraZeneca for tumor-targeted therapy. The US Food and Drug Administration (FDA) approved its capsules in December 2014 and its tablets in August 2017. It is the first poly ADP transferase (PARP) inhibitor for cancer treatment approved by FDA. Poly ADP transferase (PARP) is a key factor in the DNA excision repair pathways, while olaparib is able to inhibit PARP enzyme activity, making the single strand of DNA cleavage unrepairable, increasing genomic instability, and further leading to cellular apoptosis. Especially olaparib has a strong killing effect on tumor cells with homologous recombination repair defects. This action mode of olaparib endows it with therapeutic potential for a variety of tumors. In addition, due to the specific inhibition of olaparib to the repair ability of DNA damage, the drug will also be able to avoid tumor resistance after chemotherapy, enhance DNA damage after chemotherapy, so as to enhance the killing effect on the tumor. At present, a plurality of phase III clinical studies are conducting in AstraZeneca to investigate the treatment of olaparib for cancers associated with BRCA gene mutations, such as ovarian cancer, stomach cancer, and breast cancer.

As is known to all, in the traditional tumor chemotherapy mode, due to the lack of specific therapeutic targets, anti-tumor drugs will seriously damage the normal cells of the human body while killing of tumor cells, which will bring greater toxic side effects to the body. Targeted therapy, also known as "biological missile", has attracted a great deal of interest from researchers and medical workers in recent years due to its high specificity, less side effects, and good efficacy. Most tumors are accompanied by DNA damage or homologous recombination repair defects during the process of proliferation. Olaparib can specifically inhibit the growth of tumor cell lines, increase the toxicity on tumor cells and anti-tumor activity, and has no killing effect on tissue cells with normal DNA repair function. It is a typical drug for tumor targeted therapy so far. The emergence of Olaparib provides a treat weapon and a survival opportunity to the patients with advanced tumors carrying BRCA1 and BRCA2 mutations, as well as an example of a deeper understanding of the underlying pathogenesis of the disease and individualized treatment.

According to the results of preclinical and clinical pharmacodynamic data researched and reported by AstraZeneca, it can be seen that the steady-state plasma concentration of olaparib in vivo is directly related to its enzyme inhibitory activity and side effects. A research on a mouse model of breast cancer revealed that the effect of reducing tumor volume can only be achieved when the blood exposure continues to be greater than 50% PARP enzyme inhibition level (IC₅₀ value) for more than 13 h or greater than IC₉₀ value for more than 6 h (FDA reviews, NDA 3663410). Results obtained by AstraZeneca's mechanism model method showed a significant increase in single-strand break and clinical activity when the steady-state free plasma concentration reached above IC₉₀. However, the immediate release capsule preparation of olaparib is usually rapidly peaked within 0.5 to 3 h after oral administration and is rapidly eliminated. Therefore, in order to achieve a higher plasma concentration (>IC₉₀ value) and exert a strong enzyme inhibition effect, the 50 mg standard immediate release capsule preparation developed by AstraZeneca needs to be administrated at a dose of higher than 200 mg/2 times/day, in order to maintain at a level above IC₉₀ for a long time to achieve the desired PARP enzyme inhibitory activity, while the steady-state plasma concentrations at the dose of 100 mg/2 times/day or 400 mg/1 times/day are difficult to maintain at the level above IC₉₀ for a long time. The results of the phase II clinical efficacy study also showed that, the patient under the dose of 400 mg/2 times/day showed a quantitative advantage in the progression-free survival comparing to those under the dose of 200 mg/2 times/day, the progression-free survival for the former is 8.8 months, while for the latter is 6.5 months; and, in addition, the overall effective rate for the former is 33%, while for the latter is 25%. Although there are not statistically significant differences in therapeutic effects for the two modes of administration, considering the quantitative advantages shown by the experimental results, AstraZeneca finally chose the dose of 400 mg/2 times/day for new drug application and the marketing of capsule products. In addition, AstraZeneca's Olaparib tablets newly marketed in 2017 has a dose of 300mg/2 times/day.

However, the immediate release preparation of olaparib still shows many limitations in the process of research and development, production and clinical application.
1) Severe plasma concentration peak and valley fluctuation, and obvious dose-limiting toxicity. Although the immediate release preparation can immediately reach the plasma concentration level required for PARP enzyme inhibition, it is eliminated faster in vivo. A high oral dose (capsule 400 mg, BID or tablets 300mg, BID) is required in order to maintain the plasma concentration level required for effective enzyme inhibition for a long period, which results in a large fluctuation range of steady-state plasma concentrations of olaparib (400 mg capsules, BID, C_{min,ss} <1 µg/ml; C_{max,ss}>6 µg/mL; 300 mg tablets, BID, C_{min,ss} ∼ 1 µg / ml, C_{max,ss} > 7 µg / mL), and a steady-state plasma concentration peak which is several times or even ten times higher than the PARP enzyme IC₉₀ value, generating a lot of serious toxic side effects, including common toxic side effects such as nausea, fatigue, vomiting, diarrhea, taste disorders, and serious side effects such as myelodysplastic syndrome, acute myeloid leukemia and pneumonia.
2) Low bioavailability, requiring large administration dose. The bioavailability of the commercially available capsule is about 10 to 20% with a daily dose of 800 mg. Although the bioavailability of the tablet is improved to some extent, the daily dose is still 600 mg. Since the efficacy of the drug is related to the maintenance time of the enzyme inhibition rate, a large dose is required in order to maintain the plasma concentration of free drug required for PARP enzyme inhibition, and thus the drug utilization rate is low.
3) Inconvenient clinical administration and high cost. The high oral dose of the drug (400 mg dose, eight 0# capsules, BID) also leads to high cost in production, packaging, storage and transportation of the drug, and poor patient compliance.

In order to further improve the clinical therapeutic effect of olaparib for tumors and reduce the side effects of the drug, it is necessary to provide an excellent preparation that can precisely regulate the plasma concentration and fluctuation range of olaparib.

The patents related to the preparations of olaparib include: olaparib nanoparticle preparation (WO2015031536A1), olaparib solid dispersion and its common tablets and capsules (CN104434809A), olaparib solid dispersion and its tablets (WO2010041051, CN102238945A), which are specifically as follows.
1) WO2015031536A1 discloses an olaparib nanoparticle preparation, wherein the olaparib cationic liposomes of the invention have a particle size of 80 to 200 nm, and after loaded with a drug (or even modified with PEG), they are placed in a degradable polymeric matrix to deliver the drug in the form of an injection. Compared with oral olaparib, the olaparib nanoparticle preparation prolongs the plasma concentration half-life of the drug, and at the same time, enters the blood vessels of a tumor tissue in the form of nanoparticles, and slowly releases the drug, thereby achieving sustained release function.
2) CN104434809A discloses an olaparib solid dispersion, wherein the olaparib solid dispersion of the invention is one prepared by using povidone K 30 as a main material via solvent evaporation, spray drying or hot melt extrusion, and it may be then mixed and compressed with other fillers, lubricants, or prepared into a capsule preparation.
3) WO2010041051 A1 discloses an olaparib solid dispersion preparation which effectively improves the bioavailability of olaparib by preparing the solid dispersion preparation.

As can be seen from the above patent search results, there is no relevant research on oral sustained and controlled release preparation of olaparib.

US 2016/0008473 discloses a pharmazeutical formulation comprising olaparib in a solid dispersion with a matrix polymer that exhibits low hygroscopicity and high softening temperature.

### Disclosure of the invention

The absorption of the immediate release preparation of olaparib is not ideal, and multiple high doses are required, resulting in a high steady-state plasma concentration peak and a large plasma concentration fluctuation (400 mg, BID, C_{min,ss}<1µg/ml; C_{max,ss}>6 µg/mL; 300mg tablets, BID, C_{min,ss}∼1µg/ml, C_{max,ss}>7µg/mL), and thus the olaparib immediate release preparation has an uncontrollable clinical efficacy and a number of security issues.

The primary object of the present invention is to control the release behavior of olaparib according to its biological properties and the efficacy and safety requirements of clinical treatment, so as to precisely control the absorption rate and absorption time of olaparib in the gastrointestinal tract, control the in vivo plasma concentration level and its fluctuation range, maintain the in vivo steady-state plasma concentration at a level effective for inhibiting PARP enzyme in a long term, improve the anti-tumor effect of olaparib, and reduce the adverse reactions after administration.

Another object of the present invention is to provide an excellent preparation that minimizes the size and/or amount of tablets or capsules required for a therapeutically effective dose, with a frequency as low as possible, and improve patient compliance.

In order to achieve the above object, the present invention provides an olaparib oral sustained and controlled release pharmaceutical composition, which has controllable in vivo absorption behavior, plasma concentration and PARP enzyme inhibition level, and has advantages in improving olaparib loading and/or oral absorption and/or bioavailability and/or plasma concentration control and/or enzyme inhibition level control, and thus it can be used as the sole preparation or in combination with other therapies to treat cancer.

The olaparib oral sustained and controlled release pharmaceutical composition of the invention comprises: an olaparib in an improved dissolution form; and a release rate adjusting matrix polymer (also referred to as a release regulator), wherein the olaparib oral sustained and controlled release pharmaceutical composition has a steady-state plasma concentration valley value C_{min,ss} of 0.2 to 4 µg/mL; and a steady-state plasma concentration peak value C_{max,ss} of 0.8 to 15 µg/mL, wherein the olaparib in an improved dissolution form is selected from the group consisting of an olaparib salt, an olaparib co-grinding mixture, an olaparib nanocrystal and an olaparib solid dispersion; and the release rate adjusting matrix polymer is selected from the group consisting of a cellulose derivative, a starch or a derivative thereof, an alginate, an acrylic or methacrylic acid derivative, a polyethylene oxide, a gum, and a carbohydrate-based polymer, preferably, one or a combination of two or more selected from the group consisting of hydroxypropyl cellulose, polyethylene glycol, hypromellose, methylcellulose, hydroxyethylcellulose, ethylcellulose, cellulose acetate, sodium alginate, povidone, copolyvidone, acrylic resin, carbomer, preferably one or a combination of two or more selected from the group consisting of hydroxypropylcellulose, sodium alginate, hypromellose, and carbomer; preferably, the olaparib salt is selected from the group consisting of hydrochloride, besylate, sulfate, maleate, and camphorate; preferably, the olaparib co-grinding mixture consists of the pharmaceutically active ingredient olaparib, a matrix polymer for solubilization and other additives, and is prepared by co-grinding the components; in the co-grinding mixture, based on the total weight of the co-grinding mixture, the olaparib is 5 to 60 wt%, preferably 20 to 40 wt%, and the matrix polymer for solubilization is 40 to 95 wt%, preferably 40 to 80 wt%, the other additives is 0 to 15 wt%, preferably 0.2 to 10 wt%; preferably, the olaparib nanocrystal consists of the pharmaceutically active ingredient olaparib, a matrix polymer for solubilization, and/or other additives, and is obtained by prepared the components into nanosized particles by high pressure homogenization or coprecipitation; in the olaparib nanocrystal, based on the total weight of the olaparib nanocrystal, the olaparib is 10 to 99 wt%, preferably 20 to 50 wt%; the matrix polymer for solubilization is 1 to 75 wt%, preferably 1 to 65 wt%, and the other additives are 0 to 10 wt%, preferably 0 to 5 wt%; preferably the nanocrystal has a particle size of 50 to 1000 nm; preferably, the solid dispersion consists of the pharmaceutically active ingredient olaparib, a matrix polymer for solubilization and other additives, and is prepared by solvent evaporation or melt extrusion, in the solid dispersion, based on the total weight of the solid dispersion, olaparib is 5 to 50 wt%, preferably 10 to 40 wt%, more preferably 20 to 40 wt%, the matrix polymer for solubilization is 45 to 95 wt%, preferably 50 to 80 wt%, and the other additives are 0 to 12 wt%, preferably 0 to 10 wt%; preferably, the matrix polymer for solubilization is one or a combination of two or more selected from the group consisting of povidone, copovidone, polyoxyethylene, Soluplus, hypromellose phthalate, hydroxypropylcellulose succinate acetate, polyethylene glycol, poloxamer, polymethacrylic acid, polyethyl acrylate, 2-hydroxypropyl-β-cyclodextrin, hypromellose, polymethacrylate, hydroxypropyl cellulose, cellulose acetate phthalate, and other pharmaceutically acceptable polymers for solubilization; preferably, the other additives are one or a combination of two or more selected from the group consisting of pharmaceutically acceptable surfactants, a lubricant, a colloidal silica, a plasticizer, wherein the surfactant is preferably polyethylene glycol stearate or sodium lauryl sulfate. In addition, according to the prepared dosage form, a semi-permeable controlled release coating material, a seal coating material, a disintegrating agent, a coating powder, a plasticizer, a porogen, an expansive material, a filler, an osmotic pressure regulator (also known as osmotic agents), lubricants, adhesives (also known as binders), dyes (also known as colorants), anti-adherents (also known as anti-sticking agents), opacifiers , diluents and/or other pharmaceutically acceptable additives may also be included.

The pharmaceutically active ingredient olaparib in the olaparib pharmaceutical composition of the invention is an insoluble drug, and thus it is firstly subjected to solubilization treatment to prepare a solubilized composition, so as to improve dissolution of the drug. The solubilization treatment can be performed by chemical means to prepare a salt form compound of; or by mixing olaparib with a matrix polymer which can improve drug solubility, to change the dispersion specific surface area in the powder of the active drug preparation composition, thereby improving the dissolution properties of the drug. The solubilization treatment may include co-grinding, high pressure homogenization, coprecipitation, solvent evaporation or melt extrusion.

The olaparib in an improved dissolution form is an olaparib salt (which may be selected from the group consisting of hydrochloride, besylate, sulfate, maleate, camphorate,), an olaparib co-grinding mixture, an olaparib nanocrystal and an olaparib solid dispersion. The olaparib co-grinding mixture, olaparib nanocrystal and olaparib solid dispersion can improve the solubility and dissolution properties of olaparib in the controlled release dosage form while improving the absorption and bioavailability of the drug. The release rate adjusting matrix polymer is selected from the group consisting of a cellulose derivative, a starch or a derivative thereof, an alginate, an acrylic or methacrylic acid derivative, a polyethylene oxide, a gum, and a carbohydrate-based polymer.

Preferably, the olaparib co-grinding mixture consists of the pharmaceutically active ingredient olaparib, a matrix polymer for solubilization and other additives, and may be prepared by co-grinding the ingredients. The particle size of the mixture powder is generally sufficiently ground to less than 100 microns. The co-grinding can increase the dispersion specific surface area of the drug in the solid preparation powder, thereby improving the dissolution properties of the drug.

In the co-grinding mixture, based on the total weight of the co-grinding mixture, olaparib is 5 to 60 wt%, preferably 20 to 40 wt%, the matrix polymer for solubilization is 40 to 95 wt%, preferably 40 to 80 wt%, and other additives are 0 to 15 wt%, preferably 0.2 to 10 wt%. The total amount of the above various components is 100 wt%.

Preferably, the olaparib nanocrystal of the present invention consists of the pharmaceutically active ingredient olaparib, a matrix polymer for solubilization, and/or other additives, and is obtained by preparing the components into nanosized particles by high pressure homogenization or coprecipitation. The high pressure homogenization method may be performed as follows: a crude crystal suspension is prepared by high-speed shearing of an aqueous solution of the pharmaceutically active ingredient olaparib and the matrix polymer for solubilization, and then is added to a high pressure homogenizer, and the high pressure homogenization is performed several times, until the prepared crystal particles reached 1000 nm or less, the sample is lyophilized to prepare a uniformly dispersed olaparib nanocrystal powder. The coprecipitation method may be performed as follows: the pharmaceutically active ingredient olaparib is first dissolved in a small amount of an organic solvent such as acetone, rapidly added to a large amount of an aqueous solution in which the matrix polymer for solubilization is dissolved, and ultrasonically treated with a ultrosonic probe at high frequency (a power of 100w or more) to ensure the formation and uniform dispersion of the crystal nucleus of the pharmaceutically active drug, until a stable dispersion of the nanocrystal solution is formed, and the sample is lyophilized to prepare a uniformly dispersed olaparib nanocrystal powder. By prepared into the nanocrystals, the dispersed particle diameter of the pharmaceutically active ingredient olaparib in the solid powder can be reduced, and the specific surface area of the active drug is remarkably improved, thereby improving the dissolution property of the drug. The nanocrystal can increase the dispersion specific surface area of olaparib in the solid preparation composition powder, thereby improving the dissolution properties of the drug.

In the olaparib nanocrystal, based on the total weight of the olaparib nanocrystal, olaparib is 10 to 100 wt%, preferably 20 to 50 wt%; the matrix polymer for solubilization is 0 to 75%, preferably 0 to 65%, and other additives are 0 to 10 wt%, preferably 0 to 5 wt%. The total amount of the above various components is 100 wt%. The nanocrystal composition preferably has a particle size of 50 to 1000 nm.

The solid dispersion in the present invention preferably consists of the pharmaceutically active ingredient olaparib, a matrix polymer for solubilization, and other additives. In the solid dispersion, based on the total weight of the solid dispersion, olaparib is 5 to 50 wt%, preferably 10 to 40 wt%, more preferably 20 to 40 wt%, and the matrix polymer for solubilization is 45 to 95 wt%, preferably 50 to 80 wt%, other additives are 0 to 12 wt%, preferably 0 to 10 wt%. The total amount of the above various components is 100 wt%. The solid dispersion can be prepared by solvent evaporation or melt extrusion. The solvent evaporation method may be carried out by dissolving the drug olaparib, the matrix polymer for solubilization and/or other additives into a volatile organic solvent or organic mixed solvent, and volatilizing the organic solvent under reduced pressure, and drying in a vacuum drying oven to give a solid dispersion of olaparib. The melt extrusion method may be carried out by directly adding a uniformly mixed mixture of the drug olaparib, the matrix polymer for solubilization and/or other additive powders into a melt extruder, and collecting the extrudate of the melt. The solid dispersion enables the pharmaceutically active ingredient olaparib to be in a high-energy-state solid dispersion state, in which olaparib is dispersed in molecular level in the solid powder of the preparation composition, thereby maximizing the specific surface area of the drug. This improves the dissolution properties of the drug.

In the olaparib co-grinding mixture, olaparib nanocrystals and olaparib solid dispersion of the invention, the matrix polymer for solubilization refers to a polymer capable of stabilizing and/or solubilizing olaparib particles or molecules, and preferably may be one or a combination of two or more selected from the group consisting of povidone, copovidone, polyoxyethylene, Soluplus, hypromellose phthalate (HPMCP), hydroxypropylcellulose succinate acetate, polyethylene glycol, poloxamer, polymethacrylic acid, polyethyl acrylate, 2-hydroxypropyl-β-cyclodextrin, hypromellose (HPMC), polymethacrylate, hydroxypropyl cellulose, cellulose acetate phthalate (CAP), and other pharmaceutically acceptable polymers for solubilization.

In the olaparib co-grinding mixture, olaparib nanocrystals, and olaparib solid dispersion of the present invention, the other additives may be one or a combination of two or more selected from a common pharmaceutically acceptable surfactant for solubilization (e.g., polyethylene glycol stearate, sodium lauryl sulfate), a lubricant, colloidal silica, and a plasticizer.

The release rate adjusting matrix polymer (hereinafter sometimes referred to as a release regulator) in the present invention is a high molecular polymer having a release rate adjusting effect, which is a sustained release matrix material well known to those skilled in the art and may be one or a combination of two or more selected from a cellulose derivative, a starch or a derivative thereof, an alginate, an acrylic or methacrylic acid derivative, a polyethylene oxide, a gum, and a carbohydrate-based polymer, for example, may be one or a combination of two or more selected from the group consisting of hydroxypropyl cellulose, hypromellose, methylcellulose, hydroxyethylcellulose, ethylcellulose, cellulose acetate, sodium alginate, povidone, copolyvidone, acrylic resin, carbomer, preferably one or a combination of two or more selected from the group consisting of hydroxypropylcellulose, sodium alginate, hypromellose, and carbomer.

In the olaparib co-grinding mixture, olaparib nanocrystals and olaparib solid dispersion of the invention, the pharmaceutically active ingredient olaparib includes olaparib free base and a pharmaceutically acceptable salt thereof, and the pharmaceutically acceptable salts may be selected from the group consisting of hydrochloride, phosphate, sulfate, maleate, D(+)-camphorate and benzenesulfonate.

The olaparib oral sustained and controlled release pharmaceutical composition of the invention preferably comprises 50 to 900 parts by weight, more preferably 80 to 700 parts by weight, most preferably 120 to 600 parts by weight, of an olaparib in an improved dissolution form; and preferably 0.1 to 300 parts by weight, more preferably 20 to 250 parts by weight, most preferably 50 to 180 parts by weight, of the release rate adjusting matrix polymer. More particularly, it preferably comprises, (a) 50 to 600 parts by weight of an olaparib salt, and 10 to 250 parts by weight of a release rate adjusting matrix polymer; or (b) 50 to 700 parts by weight of an olaparib co-grinding mixture, and 10 to 200 parts by weight of a release rate adjusting matrix polymer; or (c) 50 to 800 parts by weight of olaparib nanocrystals, and 0.1 to 250 parts by weight of a release rate adjusting matrix polymer; or (d) 50 to 900 parts by weight of an olaparib solid dispersion, and 20 to 300 parts by weight of a release rate adjusting matrix polymer.

The olaparib oral sustained and controlled release pharmaceutical composition of the invention may further comprise 1 to 400 parts by weight, preferably 2 to 300 parts by weight, more preferably 5 to 250 parts by weight, of other additives. In particular, in the above (a), it may further comprise 1 to 300 parts by weight of other additives, and in the above (b), it may further contain 1 to 150 parts by weight of other additives, and in the above (c), it may further contain 1 to 200 parts by weight of other additives, and in the above (d), it may further contain 1 to 200 parts by weight of other additives.

The other additives may be selected from pharmaceutical excipients such as release rate adjusting permeation-promoting polymers, semi-permeable controlled release coating materials, seal coating materials, solubilizers, disintegrants, coating powders, plasticizers, porogens, expansive materials, fillers, osmotic pressure regulators (also known as osmotic agents, osmotic pressure promotors), lubricants, adhesives (also known as binders), dyes (also known as colorants), anti-adherents (also known as anti-sticking agents), opacifiers, diluents, and/or other pharmaceutically acceptable additives.

The expected total dose of olaparib that a body needs to take daily is 100 to 1400 mg. The amount of the pharmaceutically active ingredient olaparib contained in a single finished tablet or capsule is not particularly limited and may be selected as needed, and may be, for example, 20 to 400 mg or 50 mg to 300 mg. Preferably, the in vivo absorption rate and time of olaparib can be controlled to maintain the effective range of in vivo plasma concentration for inhibition of PARP enzyme by administering the composition once daily. The pharmaceutical composition of the present invention can enhance the PARP enzyme inhibitory and tumor therapeutic effects of olaparib, while reducing the toxic side effects of the drug.

The olaparib pharmaceutical composition of the present invention may be a sustained release preparation containing a single sustained release phase, or an immediate and sustained double release preparation containing both an immediate release phase and a sustained release phase.

The sustained release phase (also referred to as controlled release phase) is a controlled release composition containing a pharmaceutically active ingredient. The controlled release phase is preferably selected from a controlled release tablet, a controlled release pellet, a controlled release composition in a tablet, a controlled release composition in a tablet or pellet core, a controlled release layer composition incorporated into a double-layer tablet, and any combination thereof.

The immediate release phase is an immediate release composition containing a pharmaceutically active ingredient. The immediate release phase is preferably selected from the group consisting of an immediate release tablet, an immediate release pellet, an immediate release composition in a tablet, an immediate release coat layer wrapping around a controlled release tablet or pellet core, and an immediate release layer composition in a double-layer controlled release tablet and any combination thereof.

The immediate and sustained double-release preparation comprises both a sustained release phase and an immediate release phase. In the immediate and sustained double-release preparation, the pharmaceutically active ingredient in the immediate release phase preferably accounts for 10 to 50 wt%, more preferably 20 to 40 wt% of the total amount of the pharmaceutically active ingredient; the active ingredient in the sustained release phase preferably accounts for 50 to 90 wt%, more preferably 60 to 80 wt% of the total amount of the pharmaceutically active ingredient.

The olaparib pharmaceutical composition of the invention may be a tablet or a capsule, preferably selected from the group consisting of an osmotic pump controlled release tablet, an osmotic pump immediate and sustained double-release tablet, a matrix type sustained release tablet, a matrix type immediate and sustained double-release double layer tablet, a matrix type immediate and sustained double release coated tablet, a sustained release tablet based on sustained release pellets, an immediate and sustained double-release tablet based on sustained release pellets and immediate release pellets, a capsule containing matrix type sustained release pellets, a capsule containing sustained release coated pellets, a capsule containing sustained release pellets with immediate release coat, an immediate and sustained double-release capsule containing immediate release pellets and matrix type sustained release pellets, an immediate and sustained double-release capsule containing immediate release pellets and coated sustained release pellets, a capsule containing matrix type sustained release tablets, a capsule containing matrix type sustained release tables with immediate release coat, and a capsule containing immediate release tablets and matrix type sustained release tablets.

The olaparib pharmaceutical composition of the invention can be used for preparing a medicament for preventing or treating a tumor, preferably, the tumor is selected from various types of tumors with defects in DNA repair function, in particular for preparing a medicament for preventing or treating a combination of two or more cancers related to BRCA gene mutation, such as ovarian cancer, gastric cancer, breast cancer, and for preparing a medicament for preventing or treating a tumor associated with BRCA1 and BRCA2 gene mutation.

The olaparib pharmaceutical composition of the present invention has a controlled release behavior, that is, the release behavior and release amount thereof are controllable in a predetermined period of time in a release medium meeting sink conditions. When the release behavior is measured in a buffer solution with a pH of 1.2 to 7.8 at 37 °C using the apparatus II of the dissolution test method in the Chinese Pharmacopoeia, the release amount in 1 h is less than 50%, preferably less than 40%, more preferably 10 to 30% of the total amount of olaparib; the release amount in 16 h is greater than 80%, preferably >90% of the total amount of olaparib.

The olaparib pharmaceutical composition of the invention can control the absorption rate and absorption time of olaparib in the gastrointestinal tract by controlling the release behavior and the release amount. Compared with the immediate release capsule preparation, the maximum plasma concentration value (Cₘₐₓ) of olaparib of the pharmaceutical composition of the invention at the same dose is reduced by at least 10 to 70%, and the plasma concentration peak time (T_{1/2}) extends at least 30%. Through the control of plasma concentration, peak time and area under the plasma concentration-time curve, the regulation on the steady-state plasma concentration level, the fluctuation range of free plasma concentration, PARP enzyme inhibition, in vivo safety and frequency of administration of olaparib are realized.

The olaparib pharmaceutical composition of the invention can precisely regulate the in vivo steady-state plasma concentration of the drug, and has a steady-state plasma concentration valley value C_{min,ss} of 0.2 to 4 µg/mL, preferably 0.5 to 3 µg/mL, and a steady-state plasma concentration peak value C_{max,ss} of 0.8 to 15 µg/mL, preferably 1 to 12 µg/mL, and thus can achieve the **IC₉₀** value of the plasma concentration required for enzyme inhibition of cancer cells, and the steady plasma concentration peak-to-valley ratio is preferably less than 6, more preferably less than 4. A dose of 100 to 1400 mg once or twice daily can maintain a steady state plasma concentration level for effective PARP enzyme inhibition for a long time. The pharmaceutical composition of the invention can maintain, for a long-term, olaparib at the effective concentration level (the plasma concentrations for 50% and 90% PARP enzyme activity inhibition, which are respectively the **IC₅₀** and **IC₉₀** values of PARP enzyme activity) needed for the inhibition of PARP enzyme activity in vivo through regulation of the plasma concentration of olaparib, so as to accurately regulate the activity of PARP enzyme in vivo, and achieve high-efficiency and low-toxic treatment of the drug. The plasma concentration of olaparib above the **IC₅₀** and **IC₉₀** values can be maintained at least 13 h and 6 h respectively.

The sustained and controlled release pharmaceutical composition of the invention can precisely regulate the plasma concentration level and fluctuation range of olaparib, which is beneficial to the long-term maintenance of the plasma concentration level required for enzyme inhibition, and reduces the fluctuation range of plasma concentration, thereby improving the PARP enzyme inhibition rate of the tumor cells and anti-tumor efficacy and meanwhile reducing the adverse reactions of the tumor patients after administration, and increasing the compliance of the patients.

The olaparib pharmaceutical composition of the invention has the following advantages as compared with the conventional immediate release preparation:
1) It can realize the controlled release and absorption of the drug, provide accurate in vivo plasma concentration and long-term stable high level of tumor enzyme inhibition, and long lasting effect;
2) The drug absorption rate can be controlled, the plasma concentration range can be adjusted, the fluctuation of plasma concentration is small, and the adverse reactions of the patient medication are reduced;
3) It can be administered once daily, which reduces the cumbersome process of common preparations and is more convenient for clinical use;
4) Due to the controllable plasma concentration and fluctuation range, the safety window is large, and the dose and dosage regimen can be flexibly adjusted during clinical treatment.
5) The size and/or quantity of tablets or capsules required for effective therapeutic dose is minimized, which improves patient compliance, while facilitating production, storage and transportation, and increasing commercial value.

In order to better illustrate the properties of the olaparib pharmaceutical composition of the present invention, the following description is a detailed description of the present invention.

### 1. tablets

The olaparib sustained and controlled release tablet of the present invention may be an osmotic pump type controlled release tablet, a matrix type controlled release tablet or a sustained and controlled release tablet based on sustained release pellets. Among them, the osmotic pump type controlled release tablets include osmotic pump controlled release tablets and osmotic pump immediate and sustained double-release tablets, and the matrix type controlled release tablets include matrix type sustained release tablets, matrix type immediate and sustained double-release double layer tablets and matrix type immediate and sustained double-release coated tablets. The sustained and controlled release tablets based on sustained release pellets include sustained release tablets based on sustained release pellets, and immediate and sustained double-release tablets based on sustained release pellets and immediate release pellets. The sustained and controlled release tablet described above can specifically achieve the drug release behavior of the present invention in the following manners.

### 1.1 osmotic pump type controlled release tablets

The osmotic pump controlled release tablet of the invention may be a single layer osmotic pump tablet, a single layer osmotic pump immediate and sustained double-release tablet, a double layer osmotic pump controlled release tablet or a double layer osmotic pump immediate and sustained double release tablet.

The double-layer osmotic pump controlled release tablet of the invention mainly comprises:
1) a controlled release drug layer, which is formed by a controlled release drug layer composition, located in a rigid film shell and adjacent to the drug release pore;
2) a push layer (also referred to as a boost layer), which is formed by a push layer composition, located in a rigid film shell, and away from the side of the drug release pore;
3) an optional seal coat layer located between the inner surface of the rigid film shell and the core composed of the drug layer and the push layer, and prepared from the seal coating composition by drying;
4) a rigid film shell having moisture permeability, which is obtained by drying a controlled release coating solution and has one or more drug release pores at one end of the film shell;
5) an optional non-limiting aesthetic outer coat;
6) an optional non-limiting immediate release drug layer formed by an immediate release drug composition, located outside the rigid film shell/or the optional aesthetic outer coat.

Based on the total weight of the osmotic pump controlled release tablets, olaparib accounts for 3 to 50 wt% of the total weight of the osmotic pump controlled release tablets.

The controlled release drug layer composition comprises: 50 to 600 parts by weight, preferably 80 to 500 parts by weight, more preferably 120 to 400 parts by weight of an olaparib in an improved dissolution form; 10 to 150 parts by weight, preferably 20 to 120 parts by weight, more preferably 30 to 100 parts by weight of a release adjusting agent, and 0 to 40 parts by weight, preferably 0 to 30 parts by weight, of other pharmaceutically acceptable excipients.

The olaparib in an improved dissolution form may be selected from the above olaparib salt, co-grinding mixture, nanocrystals or solid dispersion of olaparib.

The release regulator may be one or combination of two or more selected from the group consisting of povidone, copovidone, polyethylene oxide, carbomer, hypromellose, croscarmellose sodium, hydroxypropyl cellulose, sodium dodecyl sulfate.

The other pharmaceutically acceptable excipients of the controlled release drug layer composition are, without limitation, selected from the group consisting of osmotic agents, lubricants, and colorants commonly used in pharmaceutical tablets, and the amounts thereof are conventionally selected in the art. The osmotic agent is one or a combination of two or more selected from the group consisting of sodium chloride, lactose, mannitol, glucose, sucrose, and fructose, preferably sodium chloride, and may be 0 to 20 parts by weight. The lubricant is one or a combination of two or more selected from the group consisting of sodium stearyl fumarate, magnesium stearate, colloidal silica, talc, polyethylene glycols, and magnesium sulfate, and may be 0 to 20 parts by weight. The colorant is one or a combination of two or more selected from the group consisting of iron oxide red, iron oxide yellow, iron oxide violet, and iron oxide black, and may be 0 to 10 parts by weight.

The push layer composition typically comprises a release rate adjusting permeation-promoting polymer, an osmotic pressure promoter, and other excipients.

The permeation-promoting polymer is a high molecular polymer which, in an aqueous medium, swells by absorbing water and promotes release of the drug in the drug layer. The release rate adjusting permeation-promoting polymer may be a material well known to those skilled in the art, and is one or a combination of two or more of selected from the group consisting of polyoxyethylene, hypromellose, hydroxypropylcellulose, croscarmellose sodium, crospovidone, sodium carboxymethyl starch, low-substituted hydroxypropyl cellulose, hydroxypropyl methylcellulose, hydroxypropyl cellulose, copolyvidone, carbomer, alginic acid and/or a derivative thereof, and may be used in an amount of 10 to 300 parts by weight, preferably 20 to 250 parts by weight, more preferably 50 to 180 parts by weight.

The osmotic pressure promoter is one or a combination of two or more selected from the group consisting of sodium chloride, lactose, mannitol, glucose, sucrose, and fructose, preferably sodium chloride, and may be used in an amount of 20 to 150 parts by weight, preferably 25 to 100 parts by weight.

Other excipients in the push layer composition include, without limitation, a lubricant, and a colorant, and may be used in an amount of 0.5 to 30 parts by weight, preferably 2 to 20 parts by weight. The lubricant is one or a combination of two or more selected from the group consisting of sodium stearyl fumarate and sodium stearate, and may be used in an amount of 0.2 to 15 parts by weight. The colorant is one or a combination of two or more selected from the group consisting of iron oxide black, iron oxide red, and iron oxide yellow, and may be used in an amount of 0.5 to 15 parts by weight.

The controlled release drug layer and the push layer together constitute a core of the osmotic pump controlled release tablet. The controlled release drug layer accounts for 40 to 80 wt% and the push layer accounts for 20 to 60 wt% based on the total weight of the core.

The seal coating layer can be prepared by spraying a seal coating solution onto the core and drying. The seal coating solution generally comprises a seal coating material and a solvent. The seal coating material is one or a combination of two or more selected from the group consisting of hypromellose, povidone, copovidone, hydroxyethylcellulose, hydroxypropylcellulose, polyethylene glycol, and stearic acid. The solvent includes one or a combination of two or more selected from the group consisting of ethanol, water, acetone, and isopropyl alcohol. The thickness of the seal coating layer can affect the release of the pharmaceutical preparation and can be controlled by the applied amount. Generally, the seal coating layer gains 0 to 10 wt% relative to the core.

The rigid film shell may also be referred to as a controlled release coat layer, and is formed by spraying a controlled release coating solution onto a core formed by a drug layer and a push layer, and the rigid film shell gains generally 3 to 20 wt%, preferably 5 to 15 wt% relative to the core.

The controlled release coating solution comprises 4 to 40 parts by weight, preferably 10 to 30 parts by weight, of a semipermeable controlled release coating material, 0 to 20 parts by weight of a plasticizer, and 0 to 20 parts by weight of a porogen, and 50 to 1000 parts by weight, preferably 200 to 800 parts by weight of a solvent.

The semipermeable controlled release coating material is one or a combination of two or more selected from the group consisting of cellulose acetate, ethyl cellulose, and acrylic resin.

The plasticizer is one or a combination of two or more selected from the group consisting of methyl phthalate, ethyl phthalate, dibutyl sebacate, triethyl citrate, tributyl citrate, tributyl acetylcitrate, glycerin acetate and castor oil.

The porogen is one or a combination of two or more selected from the group consisting of glycerin, povidone, copolyvidone, propylene glycol, polyethylene glycol, and a water-soluble inorganic salt.

The solvent is selected from one or a combination of two or more selected from the group consisting of acetone, water, ethanol, isopropanol, dichloromethane, and methanol.

The film shell has one or more drug release pores, which can be prepared by mechanical drilling or laser drilling. The drug release pores can have any geometric shape, such as a circle, an ellipse, a square, a triangle, with an average pore size ranging from 0.3 to 1.2 mm.

The aesthetic outer coat is prepared by spraying an aesthetic outer coating solution onto the core and drying, and may be optionally coated. The aesthetic outer coat is generally optionally coated onto a conventional double layer osmosis pump tablet. For an immediate and sustained double-release osmotic pump tablet with an immediate release phase coat, the aesthetic outer coat is rarely applied. The aesthetic outer coat can improve the appearance of the preparation to increase patient compliance and provide color identification. The aesthetic outer coating solution is conventional options in the art, including Opadry and other coating powders that can form the aesthetic outer coat and are known to those skilled in the art. Further, the aesthetic outer coating solution may further include one or more selected from the group consisting of a colorant, a plasticizer, an opacifier, an anti-adhesive agent, and a solvent. The aesthetic outer coat typically gains 0 to 10 wt% relative to the core.

The single-layer osmotic pump controlled release tablet of the invention mainly comprises a single-layer core and a controlled release coat film having at least one drug release pore, and can be prepared by uniformly mixing a prescription amount of an olaparib in an improved dissolution form, a release adjusting agent, an osmotic pressure promoter, and other pharmaceutically acceptable excipients, granulating and pressing to a single layer core; coating the controlled release coat film materials onto the core through a suspension coating method well known to those skilled in the art; punching a pore by using a laser drilling machine to give the single layer osmotic pump controlled release tablet. The olaparib in an improved dissolution form, the release adjusting agent, and the osmotic pressure promoter are same as the description for the double-layer osmotic pump tablet. The other pharmaceutical excipients including permeation-promoting polymers, controlled release coats, lubricants, and colorants, are same as the description for the double layer osmotic pump tablet. In the single-layer osmotic pump controlled release tablet, based on the total weight of the single-layer core, the single-layer core comprises 50 to 700 parts by weight, preferably 80 to 600 parts by weight, more preferably 120 to 400 parts by weight, of the olaparib in an improved dissolution form; 10 to 150 parts by weight, preferably 20 to 120 parts by weight, more preferably 30 to 100 parts by weight of the release adjusting agent, and 1 to 400 parts by weight, preferably 1 to 300 parts by weight, of other pharmaceutically acceptable excipients. The porogen in the sustained release coat film is 0 to 30 wt% based on the total weight of the sustained release coat film. Based on the total weight of the single-layer osmotic pump controlled release tablet, the controlled release coat film gains 3 to 30 wt% of the single-layer osmotic pump controlled release tablet.

When there is an immediate release drug layer, the osmotic pump controlled release tablet is an immediate and sustained double release osmotic pump tablet. The immediate release drug layer can be prepared by spraying an immediate release drug layer composition onto the core and drying. The immediate release drug layer composition comprises: 10 to 80 parts by weight of the active ingredient olaparib, 0 to 100 parts by weight, preferably 10 to 100 parts by weight of a matrix polymer component for solubilization, 0 to 30 parts by weight of other pharmaceutically acceptable excipients and 100 to 2000 parts by weight of solvent. The matrix polymer component for solubilization is one or a combination of two or more selected from the group consisting of povidone, copovidone, Soluplus, hypromellose phthalate (HPMCP), polyethylene glycol, poloxamer, polymethacrylic acid, polyethyl acrylate, hypromellose (HPMC), polymethacrylate, and hydroxypropyl cellulose. The other pharmaceutically acceptable excipients include those commonly used in immediate release tablets and well known to those skilled in the art, such as crospovidone, microcrystalline cellulose, and pharmaceutically acceptable surfactants (for example, sodium lauryl sulfate). The solvent includes one or a combination of two or more of ethanol, acetone, and water.

For the immediate and sustained double release osmotic pump tablet, olaparib in the immediate release drug layer is 10 to 40 wt% of the total weight of olaparib in the entire double-release osmotic pump tablet, and olaparib in the controlled release drug layer is 60 to 90 wt% of the total weight of olaparib in the entire double-release osmotic pump tablet.

The preparation method of the olaparib osmotic pump controlled release tablet comprises the following steps: (1) preparation of the olaparib in an improved dissolution form; (2) preparation of the drug layer; (3) optionally, preparation of the push layer; (4) preparation of the core; (5) optionally, preparation of seal coating layer; (6) preparation of the controlled release coat film; (7) punching a pore on the controlled release coat film of the osmotic pump tablet; (8) optionally, preparation of the esthetic outer coat; (9) optionally, preparation of the immediate release drug layer. The above (2) to (9) can be carried out by conventional pressing and coating methods well known to those skilled in the art.

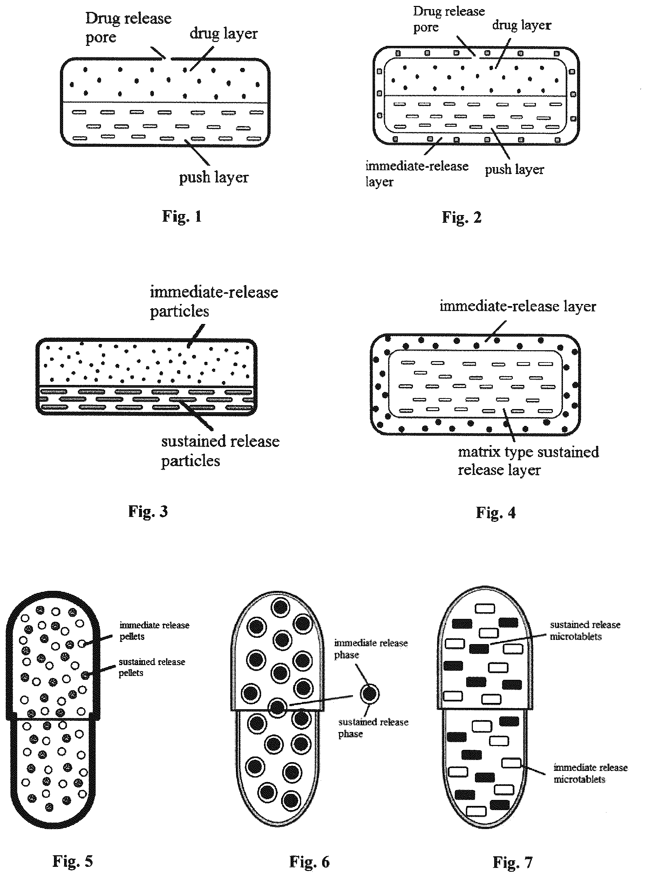

The tablet whose rigid film shell is coated with an immediate release drug layer is an osmotic pump immediate and sustained double-release tablet, and the tablet whose rigid film shell is not coated with an immediate release drug layer is a common osmotic pump controlled release tablet. Fig. 1 is a schematic view showing the structure of an osmotic pump type controlled release tablet according to an embodiment of the present invention, and Fig. 2 is a schematic view showing the structure of an osmotic pump type immediate and sustained double release tablet according to an embodiment of the present invention.

The design of the immediate and sustained double-release tablets can better exert the efficacy of olaparib, because the design of the immediate release phase ensures the initial immediate release of the drug, so as to rapidly reach the plasma concentration level required for PARP enzyme inhibition and immediately acts, and the design of the sustained release phase can ensure the sustained release of the active ingredient in the later period, ensuring the long-term maintenance of the plasma concentration required for effective enzyme inhibition, thereby maintaining the enzyme activity inhibition, improving the therapeutic effect, and reducing the toxic side effects caused by large plasma concentration fluctuation.

### 1.2. matrix type controlled release tablets

The invention also provides an olaparib controlled release matrix type tablet and/or a matrix type tablet having an immediate and sustained double release behavior,

The controlled release matrix type tablet of the invention mainly consists of a sustained release phase and an optional immediate release phase.

The double-layer tablet composed of the sustained release phase and immediate release phase is an immediate and sustained double release matrix type tablet, and the single-layer tablet composed only of the sustained release phase is an ordinary sustained release matrix type tablet. Fig. 3 is a schematic view showing the structure of a matrix type immediate and sustained double release double-layer tablet according to an embodiment of the present invention, and Fig. 4 is a schematic view showing the structure of a matrix type immediate and sustained double release coated tablet according to an embodiment of the present invention.

The sustained release phase comprises 100 to 900 parts by weight, preferably 150 to 700 parts by weight, more preferably 200 to 600 parts by weight, of the above olaparib in an improved dissolution form, 10 to 300 parts by weight, preferably 30 to 150 parts by weight of a release rate adjusting matrix polymer, 0 to 50 parts by weight of a diluent, and 0.2 to 30 parts by weight, preferably 1 to 30 parts by weight, of other common additives for tablets. The sustained release phase was prepared by thoroughly mixing the components and pressing through common methods well known for those skilled in the art.

The release rate adjusting matrix polymer may be one or a combination of two or more selected from the group consisting of polyoxyethylene, hydroxypropyl cellulose, hypromellose, methyl cellulose, hydroxyethyl cellulose, ethyl cellulose, sodium alginate, povidone, copolyvidone, acrylic resin, carbomer; preferably one or a combination of two or more selected from the group consisting of hydroxypropylcellulose, sodium alginate, hypromellose, and carbomer.

The diluent is one or a combination of two or more selected from the group consisting of microcrystalline cellulose, pregelatinized starch, sucrose, mannitol, sorbitol, sucrose, starch, sodium carboxymethyl starch. The other common additives for tablets include one or a combination of two or more kinds of lubricants, colorants, which are commonly used in solid preparations and well known to those skilled in the art. The lubricant is one or a combination of two or more selected from the group consisting of magnesium stearate, stearic acid, sodium stearyl fumarate, talc and colloidal silica. The colorant is one or a combination of two or more selected from the group consisting of iron oxide red, iron oxide yellow, iron oxide violet, iron oxide black, and titanium oxide.

The immediate release phase may comprise the above-described olaparib in an improved dissolution form, a disintegrant, a diluent, and other common additives for tablets, or comprise olaparib, a matrix polymer for solubilization, and other common additives for tablets.

The immediate release phase may be prepared by thoroughly mixing the various components, and then pressing the mixture into an immediate release layer by a conventional method well known to those skilled in the art, or by simultaneously dissolving the various components, coating the solution on the sustained release phase, and drying to form an immediate release coat film.

In the immediate release phase containing an olaparib in an improved dissolution form, the olaparib in an improved dissolution form may be used in an amount of 20 to 600 parts by weight, preferably 30 to 400 parts by weight, more preferably 50 to 250 parts by weight. The disintegrant is one or a combination of two or more selected from the group consisting of crospovidone, sodium carboxymethyl starch, low-substituted hydroxypropyl cellulose, crosslinked polyvinylpyrrolidone, croscarmellose sodium, and other pharmaceutically common used disintegrators, and may be used in an amount of 5 to 90 parts by weight, preferably 10 to 50 parts by weight. The diluent is one or a combination of two or more selected from the group consisting of microcrystalline cellulose, pregelatinized starch, sucrose, mannitol, sorbitol, sucrose, starch, sodium carboxymethyl starch, and may be used in an amount of 5 to 200 parts by weight, preferably 10 to 150 parts by weight. The other common additives for tablets include one or a combination of two or more of the lubricants and colorants commonly used in solid preparations well known to those skilled in the art, and may be used in an amount of 0.2 to 30 parts by weight, preferably 1 to 30 parts by weight. The lubricant is one or a combination of two or more selected from the group consisting of magnesium stearate, stearic acid, sodium stearyl fumarate, talc and colloidal silica, and may be used in an amount of 0.1 to 20 parts by weight. The colorant is one or a combination of two or more selected from the group consisting of iron oxide red, iron oxide yellow, iron oxide violet, iron oxide black, and titanium oxide, which may be used in an amount of 0 to 13 parts by weight.

In an immediate release phase comprising olaparib, a matrix polymer for solubilization and other common additives for tablets, the amount of olaparib may be 5 to 100 parts by weight, preferably 10 to 80 parts by weight, more preferably 20 to 60 parts by weight. The matrix polymer for solubilization is one or a combination of two or more selected from the group consisting of povidone, copovidone, Soluplus, hypromellose phthalate (HPCP), polyethylene glycol, poloxamer, hypromellose (HPMC) and other materials, and may be used in an amount of 5 to 300 parts by weight, preferably 20 to 200 parts by weight, more preferably 30 to 120 parts by weight. The other common additives for tablets include the common additives for immediate release tablets which are well known to those skilled in the art, such as crospovidone, microcrystalline cellulose, and pharmaceutically acceptable surfactants (such as sodium lauryl sulfate), mannitol, and lubricants (such as magnesium stearate), and may be used in an amount of 0.1 to 150 parts by weight, preferably 0.5 to 100 parts by weight.

In a matrix type immediate and sustained double release tablet, olaparib in the immediate release phase is 10 to 40 wt% of the total weight of olaparib in the entire immediate and sustained double release matrix type tablet, and olaparib in the sustained release phase is 60 to 90 wt% of the total weight of olaparib in the entire immediate and sustained double release matrix type tablet.

The olaparib controlled release preparation having the immediate and sustained release behavior according to the present invention is characterized in that, in accordance with the requirements of the release test method of Chinese Pharmacopoeia 2015, in a release medium meeting sink conditions, preferably more than 90 wt% of the pharmaceutically active ingredient in the immediate release phase is released within 2 h, more preferably more than 90 wt% of the pharmaceutically active ingredient in the immediate release phase is released within 1 h; the time for releasing more than 90 wt% of the pharmaceutically active ingredient in the sustained release phase is preferably 10 to 16 h, preferably 16 h or more; the release behavior of the pharmaceutically active ingredient in the sustained release phase complies with the zero-order, first-order, Higuchi or Ritger-Peppas release model, preferably zero-order release model.

### 1.3. Sustained release tablets based on sustained release pellets

Another aspect of the invention provides an olaparib sustained release tablet based on sustained release pellets. The olaparib sustained release tablet based on sustained release pellets may be a sustained release tablet based on sustained release pellets, or an immediate and sustained double release tablet based on an immediate release matrix / sustained release pellets.

In the immediate and sustained double release tablet, the immediate release matrix constitutes the immediate release phase, and the sustained release pellets constitute the sustained release phase. In the entire immediate and sustained double release tablet, the olaparib in the immediate release phase accounts for 10 to 40 wt% of the total amount of olaparib; the olaparib in the sustained release pellet accounts for 60 to 90 wt% of the total amount of olaparib.

The immediate release matrix may include the above-described pharmaceutically active ingredient in an improved dissolution form, a disintegrant, a diluent, and other common additives for tablets.

In an immediate release matrix comprising a pharmaceutically active ingredient in an improved dissolution form, the olaparib in an improved dissolution form may be used in an amount of 20 to 200 parts by weight, preferably 50 to 150 parts by weight. The disintegrant is one or a combination of two or more selected from the group consisting of crospovidone, sodium carboxymethyl starch, low-substituted hydroxypropyl cellulose, crosslinked polyvinylpyrrolidone, croscarmellose sodium, and other pharmaceutically common used disintegrants, and may be used in an amount of 5 to 200 parts by weight, preferably 10 to 100 parts by weight, more preferably 20 to 80 parts by weight. The diluent is one or a combination of two or more selected from the group consisting of microcrystalline cellulose, pregelatinized starch, sucrose, mannitol, sorbitol, sucrose, starch, sodium carboxymethyl starch, and may be used in an amount of 5 to 200 parts by weight, preferably 10 to 150 parts by weight. The other common additives for tablets is one or a combination of two or more selected from the group consisting of the lubricants and colorants, which are commonly used in solid preparations and well known to those skilled in the art, and may be used in an amount of 0.2 to 30 parts by weight, preferably 1 to 30 parts by weight. The lubricant is one or a combination of two or more selected from the group consisting of magnesium stearate, stearic acid, sodium stearyl fumarate, talc and colloidal silica, and may be used in an amount of 0.1 to 20 parts by weight. The colorant is one or a combination of two or more selected from the group consisting of iron oxide red, iron oxide yellow, iron oxide violet, iron oxide black, and titanium oxide, and may be used in an amount of 0 to 13 parts by weight.

The sustained release pellets may comprise coated sustained release pellets and matrix type sustained release pellets, and may be prepared, without limitation, from a blank pellet core (0 to 300 parts by weight), an olaparib salt (for example, a hydrochloride salt, benzene sulfonate, sulfate, camphorate), a release rate adjusting matrix or controlled release coating material, and other excipients, by conventional methods well known to those skilled in the art such as wet granulation, extrusion spheronization, pan coating and/or fluidized bed granulation coating. For example, the sustained release pellets are prepared by one-pot drug-loading pan coating, wherein olaparib is dispersed or loaded on a blank pellet core to form a drug-loaded pellet core, which is then coated with a layer of sustained release film materials such as Surelease or the like to form a sustained release coat film, so as to form a coated sustained release pellet. The blank pellet core is one or a combination of two or more selected from the group consisting of a sucrose pellet core, a starch pellet core, a microcrystalline cellulose pellet core, a silica pellet core, and a hydroxypropyl cellulose pellet core. For another example, the sustained release pellets are prepared by fluidized bed method, wherein olaparib and a release rate adjusting matrix are simultaneously dissolved, and then placed in a spray drying apparatus, blasted into a gas stream, and spray dried, and the collected product is added with a binder, granulated and dried to form the matrix type sustained release pellets.

In the sustained release pellets, the release rate adjusting matrix or the controlled release coating material may be one or more selected from the group consisting of shellac, cellulose acetate phthalate (CAP), acrylic resin (Eudragit), and ethyl cellulose (EC), polyacrylic polysiloxane, cellulose acetate, cellulose propionate, cellulose acetate propionate, polyvinyl alcohol, polyvinylpyrrolidone (PVP), methyl cellulose, hydroxypropyl cellulose, and hydroxypropyl group cellulose (HPMC). The other excipients mainly include a binder, a plasticizer, and a porogen. Among others, the binder is one or more selected from the group consisting of polyethylene glycol (PEG), stearic acid, glyceryl monostearate, the plasticizer is one or more selected from the group consisting of propylene glycol, glycerin, polyethylene glycol (PEG), triacetin, acetyl monoglyceride, phthalate, castor oil, and the porogen is one or more selected from the group consisting of a hydrophilic liquid carrier (glycerin, PEG200), sugars (lactose, fructose, sucrose, mannose), surfactants (polysorbate 80, sodium lauryl sulfate), polymers (povidone, hypromellose).

In one embodiment, the sustained release pellets comprise 100 to 500 parts by weight, preferably 200 to 400 parts by weight, of a blank pellet core, 10 to 150 parts by weight, preferably 30 to 100 parts by weight, of an olaparib salt, 10 to 300 parts by weight of a release rate adjusting substrate or a controlled release coating material, 0 to 100 parts by weight of a binder, 0 to 12 parts by weight of a porogen, and 0 to 15 parts by weight of a plasticizer.

Finally, the sustained release pellets were directly compressed to obtain sustained release tablets based on sustained release pellets. In the case that the immediate release matrix and the sustained release pellets are uniformly mixed at a certain ratio, and then compressed into tablets by a tableting machine with a special stirring function, an immediate and sustained double-release preparation can be prepared.

### 2. capsules

The present invention also provides a controlled release capsule preparation which may be selected from the group consisting of a pellet-based sustained and controlled release capsule and a tablet-based sustained and controlled release capsule.

### 2.1. pellet-based controlled release capsules

The pellet-based sustained and controlled release capsule of the present invention is a controlled release capsule composed of sustained release pellets, or an immediate and sustained double release capsule composed of sustained release pellets and immediate release pellets, and may include capsules containing matrix type sustained release pellets, capsules containing coated sustained release pellets, capsules containing sustained release pellets having immediate release coat, immediate and sustained double-release capsules containing immediate release pellets and matrix type sustained release pellets, and immediate and sustained double-release capsules containing immediate release pellets and coated sustained release pellets. Fig. 5 is a schematic view showing the structure of an immediate and sustained double-release capsule containing immediate release pellets and matrix type sustained release pellets, and Fig. 6 is a schematic view showing the structure of a capsule containing sustained release pellets with immediate release coat.

The pellet-based sustained and controlled release capsule of the present invention may be sustained release capsules based on sustained release pellets, or immediate and sustained double release capsules based on immediate release pellets and sustained release pellets. For the immediate and sustained double release capsules, the immediate release pellets constitute the immediate release phase, and the sustained release pellets constitute the sustained release phase. Based on the total weight of olaparib in the double-release capsule, the olaparib in the immediate release phase accounts for 10 to 40 wt%; and the olaparib in the sustained release pellets accounts for 60 to 90 wt%.

The descriptions in terms of the composition, preparation method, materials selection and amount thereof for the coated sustained release pellets and the matrix type sustained release pellets are the same as those for the sustained release pellets of the above section 1.3, which are not repeated again.

The sustained release pellets containing immediate release coat can be prepared by directly coating the surface of the above-mentioned matrix type sustained release pellets or the coated sustained release pellets with an immediate release matrix.

The immediate release pellets can be prepared by dissolving the immediate release matrix, coating it onto a blank pellet core by a conventional coating method well known to those skilled in the art, or directly preparing the immediate release matrix into pellets.

The descriptions in terms of the composition, materials selection and amount thereof for the immediate release matrix are the same as those for the immediate release matrix of Section 1.2 above, which are not repeated again.

The controlled release capsule can be prepared from the controlled release pellets by capsule filling as follows: weighing and evenly mixing the above immediate release pellets and sustained release pellets at a certain ratio, and then carrying out capsule filling to prepare an immediate and sustained double release capsule preparation, or capsule filling the sustained release pellets having an immediate release coat to prepare an immediate and sustained double release capsule preparation.

### 2.2. microtablet based controlled release capsules

The microtablet based sustained and controlled release capsules of the invention is controlled release capsules composed of sustained release microtablets or immediate and sustained double release capsules composed of sustained release microtablets and immediate release microtablets, and may include capsules containing matrix type sustained release microtablets, capsules containing matrix type sustained release microtablets with immediate release coat, and capsules containing immediate release microtablets and matrix type sustained release microtablets. In general, for the filling of hard capsules, the produced microtablets have a small diameter of typically <5 mm. Fig. 7 is a schematic view showing the structure of a capsule containing immediate release microtablets and sustained release microtablets.

For the immediate and sustained double-release capsules, the immediate release microtablets constitute the immediate release phase, and the sustained release microtablets constitute the sustained release phase. Based on the total weight of olaparib in the capsule, the olaparib in the immediate release phase is 10 to 40 wt%; and the olaparib in the sustained release phase is 60 to 90 wt%.

The descriptions in terms of composition, preparation method, materials selection and amount thereof for the matrix type sustained release microtablets are the same as those for the above-mentioned matrix type controlled release tablet of Section 1.2, which are not repeated again.

The matrix type sustained release microtablets containing immediate release coat can be prepared by directly coating an immediate release matrix onto the surface of the above matrix type sustained release microtablets.

The immediate release microtablets can be prepared by directly compressing the immediate release matrix.

The descriptions in terms of composition, preparation method, materials selection and amount thereof for the immediate release matrix are the same as those for the above-mentioned immediate release matrix of Section 1.2, which are not repeated again.

The sustained release capsule preparation can be prepared by filling the matrix type sustained release microtablets into capsules, and the immediate and sustained double release capsules can be prepared by mixing the immediate release microtablets and the sustained release microtablets at a certain ratio and filling them into capsules, or filling the matrix type sustained release microtablets with immediate release coat into capsules.

### DRAWINGS

Fig. 1 is a schematic view showing the structure of an osmotic pump type controlled release tablet;
Fig. 2 is a schematic structural view of an osmotic pump type immediate and sustained double release tablet;
Fig. 3 is a schematic structural view of a matrix type immediate and sustained double release double-layer tablet;
Fig. 4 is a schematic structural view of a matrix type immediate and sustained double-release release coated tablet;
Fig. 5 is a structural view of a capsule containing immediate release pellets and matrix type sustained release pellets;
Fig. 6 is a structural view of a capsule containing sustained release pellets with immediate release coat;
Fig. 7 is a structural view of a capsule containing immediate release microtablets and sustained release microtablets;
Fig. 8 is a graph showing the release profiles of the double-layer osmotic pump controlled release tablet of Example 1 in release media at pH 1.2, 4.5 and 6.8;
Fig. 9 is a graph showing the release profiles of the preparations of Examples 2, 5, 6, 7, 9 and 11 in a release medium at pH 6.8;
Fig. 10 is a graph showing the release profile of the matrix type sustained release tablet of Example 3 in a release medium at pH 6.8;
Fig. 11 is a graph showing the release profile of the immediate and sustained double-release matrix type tablet of Example 4;
Fig. 12 is a graph showing the dissolution profile of the immediate release capsule preparation of Comparative Example 1.
Fig. 13 is a graph showing the in vivo plasma concentration-time curves of the immediate release capsule of Comparative Example 1 and the double-layer osmotic pump controlled release tablet of Example 1;
Fig. 14 is a graph showing the in vivo plasma concentration-time curves of the immediate release capsule of Comparative Example 1 and the immediate and sustained double-release tablet of Example 4;
Fig. 15 is a graph showing the in vivo plasma concentration-time curves of the immediate release capsule of Comparative Example 1 and the immediate and sustained double-release tablet of Example 2;
Fig. 16 is a graph showing PARP enzyme inhibition rate-time curves of the immediate release capsule of Comparative Example 1 and the immediate and sustained double-release tablet of Example 2 in canine PBMC.

### Best mode for carrying out the invention

The following examples generally describe the preparation and/or characterization of typical compositions of the invention, wherein all percentages are by weight unless otherwise indicated. In the following examples, various processes and methods not described in detail are conventional methods well known in the art.

Test animals: Beagle dogs were half males and half females, weighing 8 to 10 kg, from Beijing Marshall Biotechnology Co., Ltd. The test animals were subjected to adaptive feeding at the Experimental Animal Center of Shanghai Institute of Materia Medica for 14 days before the test day.

A single-punch tablet press (TDP-1, Guangzhou Xulang Machinery Equipment Co., Ltd.) was adopted to press tablets.

The three-dimensional mixer was a T2F model available from TURBULA. The melt extruder was a Pharma 11 model available from Thermo Fisher.

### Example 1 Double-layer osmotic pump controlled release tablets

| **Components of drug laver of core** | **dose (100 tablets)** |
|---|---|
| Olaparib | 5g |
| Copolyvidone(VA64) | 10g |
| Polyvidone(K90) | 2g |
| Magnesium stearate | 0.3g |

| **Components of push layer of core** | **dose (100 tablets)** |
|---|---|
| Carboxymethyl starch sodium | 8.4g |
| Hypromellose (K15M) | 1.7g |
| Carbomer(971P) | 0.6g |
| Sodium chloride | 5.8g |
| copolyvidone (VA64) | 3.6g |
| Red ferric oxide | 0.2g |
| Magnesium stearate | 0.2g |

A solid dispersion was prepared from Olaparib and copolyvidone VA64 by solvent evaporation. That is, olaparib and copolyvidone VA64 were simultaneously dissolved in ethanol/acetone (25/75, v/v), evaporated off the organic solvent under reduced pressure, dried in a vacuum drying oven, and ground and pulverized through a 60 mesh screen to be ready for tableting. For the obtained solid dispersion, in water under sink conditions at 37 °C and 100 rpm, the pharmaceutically active ingredient was dissolved out 90% or more in 30 min. However, under the same conditions, the olaparib compound powder was dissolved out less than 60% in 2h.

Then, the solid dispersion and other excipients in prescription amounts were passed through a 60 mesh screen and mixed by a three-dimensional mixer at 30 rpm for 25 min to obtain a drug layer composition to be ready for tableting.

The push layer excipients were accurately weighed and passed through a 60 mesh screen and mixed by a three-dimensional mixer at 30 rpm for 30 min to obtain a push layer composition.

An osmotic pump double-layer core comprising a drug layer and a push layer was pressed from the above-described drug layer composition and the push layer composition by direct pressing.

The pressed core was coated with a 4% cellulose acetate solution to form a controlled release coat layer with a weight increment of 10%, so as to obtain a double-layer osmotic pump controlled release tablet.

The dissolution degree of the double-layer osmotic pump controlled release tablets was determined on the apparatus II of the dissolution test method (Chinese Pharmacopoeia 2010, second edition, appendix X, C). The measurements were operated following the method at 37 °C, using buffers of pH 1.2, 4.5, and 6.8 as the release media (7.65mL of hydrochloric acid was diluted with water to 1000mL to obtain a release medium of pH 1.2; 250mL of a 0.2mol/L potassium dihydrogen phosphate solution was added with 0mL and 112mL of a 0.2mol/L sodium hydroxide solution, to respectively obtain release media of pH 4.5 and 6.8) respectively, at a rotary speed of 75 rpm, 6 mL of the solution was taken at 0.5, 1, 2, 4, 6, 8, 10, 12, 13, 16 h, respectively, and centrifuged, the supernatant was taken as the test solution to determine the release degree.

According to the ultraviolet-visible spectrophotometry (Chinese Pharmacopoeia 2010, second edition, appendix IV, A), the absorbance was measured at a wavelength of 278 nm, so as to measure the release degree of the prescription tablet.

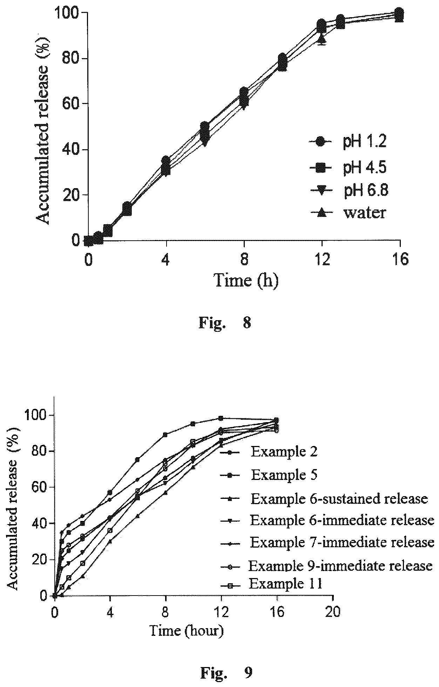

The release results in release media with different pH are shown in Fig. 8. The results showed that the double-layer osmotic pump controlled release tablets were basically not affected by pH. The active ingredient olaparib could be released at a constant rate, less than 10% in 1 h, 50% in 6 h, and more than 90% in 12 h. The overall release time can be 12 to 13 h.

### Example 2 Immediate and sustained double release double layer osmotic pump controlled release tablets

| **Components of drug layer of cores** | **dose (100 tablets)** |
|---|---|
| olaparib | 4g |
| copolyvidone(VA64) | 16.8g |
| polyvidone(K90) | 1.5g |
| sodium dodecyl sulfate | 0.5g |
| magnesium stearate | 0.3g |

| **Components of push layer of cores** | **dose (100 tablets)** |
|---|---|
| Carboxymethyl starch sodium | 7.0g |
| Hypromellose (K15M) | 1.6g |
| Carbomer(971P) | 0.5g |
| Sodium chloride | 5.0g |
| copolyvidone (VA64) | 3.0g |
| Black ferric oxide | 0.1g |
| magnesium stearate | 0.1g |

The olaparib and copolyvidone were sieved through a 60 mesh screen for 3 times, and then mixed by a three-dimensional mixer at 30 rpm for 25 min. The mixture was slowly added to a preheated melt extruder, and the extrudate was collected, pulverized, passed through a 60 mesh screen to give an olaparib solid dispersion. Then, the olaparib solid dispersion and other excipients except magnesium stearate in prescription amounts were passed through a 60 mesh screen and mixed by a three-dimensional mixer at 30 rpm for 25 min, and magnesium stearate was added and the mixing continued for 5 min to obtain a drug layer composition to be ready for tableting.

The push layer excipients were accurately weighed and passed through a 60 mesh screen and mixed by a three-dimensional mixer at 30 rpm for 30 min to obtain a push layer composition.

An osmotic pump double-layer core comprising a drug layer and a push layer was pressed from the above-described drug layer composition and the push layer composition by direct pressing.

The pressed core was coated with a 3% cellulose acetate - 0.2% PEG4000 solution to form a controlled release coat layer with weight increment of 10% to give a double-layer osmotic pump controlled release tablet.

The olaparib solid dispersion was dissolved in acetone, and the solution was coated on the obtained double-layer osmotic pump tablet in a ratio of the drug content in the immediate release layer to that in the sustained release layer of 2:8, to give an immediate and sustained double release double-layer osmotic pump tablet, in which the active ingredient accounted for 20% in the immediate release layer and 80% in the sustained release layer.

The dissolution degree of the immediate and sustained double release double-layer osmotic pump controlled release tablets was determined on the apparatus II of the dissolution test method (Chinese Pharmacopoeia 2010, second edition, appendix X, C). The measurements were operated following the method at 37 °C, using a buffer of pH 6.8 as the release medium (250mL of 0.2mol/L potassium dihydrogen phosphate solution was added with 112 mL of 0.2mol/L sodium hydroxide solution, to obtain a release medium of pH 6.8), at a rotary speed of 75 rpm, 6 mL of the solution was taken at 0.5, 1, 2, 4, 6, 8, 10, 12, 13, 16 h, respectively, and centrifuged, the supernatant was taken as the test solution to determine the release degree.

According to the ultraviolet-visible spectrophotometry (Chinese Pharmacopoeia 2010, second edition, appendix IV, A), the absorbance was measured at a wavelength of 278 nm, so as to measure the release degree of the prescription tablet.

The release results in the release medium of pH 6.8 are shown in Fig. 9. It was shown that the immediate and sustained double release double layer osmotic pump controlled release tablets could release the drug in the immediate release layer within 2 h, and the drug in the sustained release layer could be released at a constant rate, more than 80% or more of the drug can be released within 16h, and the drug releasing time is up to 16 h.

### Example 3 Sustained release matrix type tablets

| **name** | **dose (100 tablets)** |
|---|---|
| olaparib | 8g |
| Polyvidone K30 | 24g |
| hydroxy propyl cellulose (K4M) | 4g |
| sodium dodecyl sulfate | 0.2g |
| magnesium stearate | 0.2g |

Olaparib and povidone K30 were sieved through a 60 mesh screen for 3 times, and then mixed by a three-dimensional mixer at 30 rpm for 25 min. The mixture was slowly added to a preheated melt extruder, and the clear extrudate was collected, pulverized and passed through a 60 mesh screen to give an olaparib solid dispersion. The solid dispersion, hydroxypropylcellulose (K4M) as the release rate adjusting matrix polymer, sodium lauryl sulfate in prescription amounts were sieved through a 60 mesh screen and mixed in a three-dimensional mixer at 30 rpm for 25 min, and then added with a prescription amount of magnesium stearate and mixed for further 5 min, and compressed to prepare a sustained release matrix type tablet with suitable hardness.

The method for measuring the release degree of the olaparib sustained release matrix type tablets was the same as that in Example 1, using a buffer of pH 6.8 as a release medium.

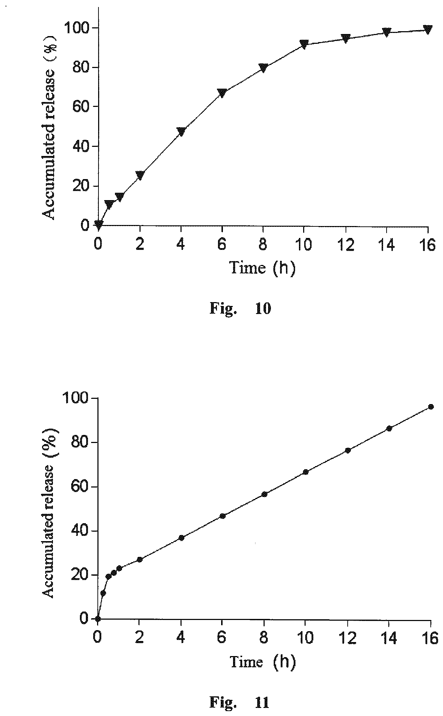

The results are shown in Fig. 10. The sustained release matrix type tablets can release less than 20% of olaparib in 1 h, about 80% in 8 h, more than 90% in 16 h, and the drug release time was up to 10 h.

### Example 4 Immediate and sustained double release matrix type double-layer tablets

| **Immediate release layer** | **dose(g) (1000 tablets)** |
|---|---|
| olaparib | 20 |
| Soluplus | 75 |
| colloidal silica | 1 |
| Crosslinked polyvidone (PVPP XL) | 10 |
| mannitol | 5 |
| magnesium stearate | 3 |

| **Sustained release layer** | **dose(g) (1000 tablets)** |
|---|---|
| olaparib | 100 |
| Polyvidone K30 | 300 |
| colloidal silica | 3.5 |
| HPMC K15M | 70 |
| magnesium stearate | 3 |

Immediate release layer: olaparib, colloidal silica and Soluplus in prescription amounts were sieved through a 60 mesh screen and mixed in a three-dimensional mixer at 30 rpm for 25 min, and then slowly added to a preheated melt extruder, and the extrudate was collected, pulverized and passed through a 60 mesh screen to give an olaparib solid dispersion.

The resulting olaparib solid dispersion was mixed uniformly with a prescription amount of other materials such as the disintegrant PVPP XL and other excipients such as mannitol and magnesium stearate to be ready for tableting.

Sustained release layer: olaparib, colloidal silica and povidone K30 in prescription amounts are sieved through a 60 mesh screen and mixed uniformly, slowly added to a preheated melt extruder, and the extrudate was collected, pulverized and passed through a 60 mesh screen to give an olaparib solid dispersion.

The resulting olaparib solid dispersion was mixed uniformly with a prescription amount of a release rate adjusting polymer HPMC K15M and the lubricant magnesium stearate to be ready for tableting.

Tableting: a direct compression method is used to prepare an immediate and sustained double release matrix type double-layer tablet with a suitable hardness.

The dissolution degree of the controlled release preparation was determined on the apparatus II of the dissolution test method (Chinese Pharmacopoeia 2010, second edition, appendix X, C). The measurements were operated following the method at 37 °C, using a buffer of pH 6.8 as the release medium, at a rotary speed of 75 rpm, 6 mL of the solution was taken at 0.25, 0.5, 0.75, 1, 2, 4, 6, 8, 10, 12, 13 and 16 h, respectively, and centrifuged, and the supernatant was taken as the test solution to determine the release degree.

According to the ultraviolet-visible spectrophotometry (Chinese Pharmacopoeia 2010, second edition, appendix IV, A), the absorbance was measured at a wavelength of 278 nm, so as to measure the release degree of the obtained tablet.

The release results were shown in Fig. 11. The immediate and sustained double release matrix type double-layer tablet can rapidly release about 20% of the drug in 30 min, and about 60% of the drug was released in about 8 h, and the drug was completely released in about 16 h. The release behavior can control the plasma concentration range of olaparib, can immediately reach the plasma concentration required for PARP inhibition after oral administration, and maintain the concentration level for a long time.

### Example 5 Sustained release matrix type coated tablets containing an immediate release coat layer

(1) Preparation of sustained release core (100 pieces)

| **name** | **dose** |
|---|---|
| olaparib | 8g |
| poloxamer 188 | 24g |
| sodium alginate | 7g |
| magnesium stearate | 0.2g |

(2) Immediate release coat

| **name** | **dose** |
|---|---|
| olaparib | 2g |
| poloxamer 188 | 5g |
| talc | 1g |
| polyethyleneglycol 4000 | 2g |
| 95% ethanol | 90g |
| water | 10g |

The preparation method was as follows.

Preparation of the sustained release core of the sustained release matrix type coated tablets: olaparib and poloxamer 188 in prescription amounts were sieved through a 60 mesh screen and mixed in a three-dimensional mixer at 30 rpm for 25 min, and then slowly added to a preheated melt extruder, the extrudate was collected, pulverized and passed through a 60 mesh screen to give an olaparib solid dispersion. The prepared olaparib solid dispersion was mixed uniformly with the dissolution rate adjusting matrix polymer sodium alginate, and then added and mixed uniformly with a lubricant magnesium stearate, and compressed by direct pressing to give a sustained release tablet core with suitable hardness.

Coating of immediate release coat: According to the prescription of the immediate release coat, an immediate release coating solution was prepared, and the sustained release tablet cores were placed in a high-efficiency coating pan for coating the immediate release coat; finally, they were dried at 45 °C for 12 h to remove excess organic solvent and water so as to give the sustained release matrix type coated tablets.

The release degree was determined in the same manner as in Example 1, using a buffer of pH 6.8 as the release medium. The release curve is shown in Fig. 9.

### Example 6 Capsules containing sustained release pellets, or immediate and sustained double-release capsules containing immediate release pellets and sustained release pellets

### (1) Sustained release pellets

I) Drug-loaded pellet cores

| **name** | **dose** |
|---|---|
| Olaparib chloride | 100g |
| microcrystalline cellulose blank pellet cores | 400g |
| hydroxy propyl cellulose SSL | 100g |
| 95% ethanol | 400ml |

II) Seal coating

| **name** | **dose** |
|---|---|
| drug loaded pellet cores | 600g |
| polyvidone (K30) | 50g |
| 95% ethanol | 200ml |

III) Sustained release coat

| **name** | **dose** |
|---|---|
| drug loaded pellet cores with seal coating | 650g |
| Surelease (aqueous dispersion) | 200ml |
| water | 200ml |

### 2 Immediate release pellets

| **name** | **dose** |
|---|---|
| olaparib chloride | 25g |
| copolyvidone (VA64) | 50g |
| microcrystalline cellulose blank pellet core | 100g |
| 95% ethanol | 300ml |

The preparation method was as follows.

Immediate release pellets: olaparib chloride and VA64 were dissolved or dispersed in a 95% ethanol solution so as to prepare a drug-loading solution, which was sprayed on a prescription amount of microcrystalline cellulose blank pellet cores by fluidized bed coating, to give immediate release pellets.

Sustained release pellets:
A release rate adjusting matrix hydroxypropyl cellulose SSL was weighed and dispersed in a 95% ethanol solution, so as to prepare a coating solution with a solid content of 10%, and stirred well on a magnetic stirrer;
A prescription amount of olaparib chloride was weighed and uniformly dispersed in the above coating solution, to be ready for being used as a drug-loading coating solution.

Microcrystalline cellulose blank pellet cores were added to a fluidized bed, and the operating parameters such as air volume and temperature were adjusted, the prepared drug-loading coating solution was sprayed to load the drug to give the drug-loaded pellet cores.

The component of the seal coating layer film was dissolved or dispersed in a 95% ethanol solution, and sprayed into a prescription amount of the drug-loaded pellet cores by fluidized bed coating to give a drug-loaded pellet cores with seal coating layer.

The aqueous dispersion of the sustained release coating solution was diluted with an appropriate amount of an aqueous solution, mixed well, and used as a coating solution for the sustained release coat film, which was sprayed onto the drug-loaded pellet cores with seal coating layer by fluidized bed coating so as to give sustained release pellets.

Capsule filling: The prepared sustained release pellets were encapsulated to prepare sustained release capsules.

The prepared immediate release pellets and sustained release pellets were thoroughly mixed in prescription amounts, and then filled into capsules to prepare immediate and sustained double-release capsules.

The release degree was determined in the same manner as that in Example 1, using a buffer of pH 6.8 as the release medium. The release curves of the sustained release capsules and the immediate and sustained double-release capsules were shown in Fig. 9, respectively.

### Example 7 Sustained and controlled release tablets based on sustained release pellets

### (1) Sustained release pellets

I) Dru-loaded pellet cores

| **name** | **dose** |
|---|---|
| olaparib | 100g |
| Sucrose blank pellet cores | 400g |
| copolyvidone (VA64) | 200g |
| 95% ethanol | 400ml |

II) Seal coatins

| **name** | **dose** |
|---|---|
| drug-loaded pellet core | 700g |
| Hypromellose (E5) | 50g |
| 95% ethanol | 200ml |

III) Sustained release coat

| **name** | **dose** |
|---|---|
| drug-loaded pellet cores with seal coating | 750g |
| Eudragit NE30D | 150g |
| talc | 5g |
| PEG 4000 | 15g |
| water | 750ml |

The preparation method was as follows:

Drug-loaded pellet cores: Olaparib and VA64 were dissolved or dispersed in a 95% ethanol solution to be formulated into a drug-loading solution, which was sprayed into a prescription amount of sucrose blank pellet cores by fluidized bed coating, to give drug-loaded pellet cores.

Sustained release pellets:
The components of the seal coating layer film were dissolved or dispersed in a 95% ethanol solution, and sprayed onto a prescription amount of drug-loaded pellet cores by fluidized bed coating to give drug-loaded pellet cores with seal coating.

The aqueous dispersion of the sustained release coating solution was added with talc and an appropriate amount of an aqueous solution and mixed well to give a coating solution for the sustained release coat film, which was sprayed onto the drug-loaded pellets cores with seal coating by fluidized bed coating to give sustained release pellets.

Sustained and controlled release tablets: microcrystalline cellulose was added into ethanol to form pellets, which were evenly mixed with the sustained release pellets, and then added with silica or magnesium stearate, uniformly mixed and then tableted.

Immediate and sustained double-release tablets: the above-prepared immediate release pellets (drug loaded pellet cores) and sustained release pellets were thoroughly mixed in prescription amounts, and then added with silica or magnesium stearate, evenly mixed, and then tableted. The release degree was determined in the same manner as that in Example 1 by using a buffer of pH 6.8 as the release medium. The release curve is shown in Fig. 9.

### Example 8 Sustained and controlled release tablets based on sustained release pellets

60g of olaparib besylate, 140g of microcrystalline cellulose, and 100g of lactose were sieved and mixed through a 80 mesh screen, and then transferred into a wet granulator, whose parameters were adjusted. A 1 wt% aqueous solution of hypromellose E15 as a binder was added to prepare a soft material, which was then extruded and spheronized to prepare olaparib-containing pellets, wherein the extrusion screen had a pore size of 0.5 mm, the extrusion speed was 20 r/min, the spheronization speed was 1000 r/min, and the drying was performed at 40 °C on the fluidized bed. The 30 to 40 mesh drug-containing pellets were sieved to be ready for use.

The sieved olaparib pellets were placed in a fluidized bed, and coated with a prepared coating solution, to prepare olaparib sustained release pellets. The formulation of the coating solution: an acrylic resin 14.5%, a plasticizer triethyl citrate 5%, an anti-sticking agent talc 10.5% and water for balance.

25g of olaparib sustained release pellets, 5g of drug-containing pellets, 12g of microcrystalline cellulose, 16g of lactose, 12g of a 5 wt% pvpK30 solution, were weighed and pelletized through a 18 mesh screen, dried in a 40°C oven, sieved through a 18 mesh screen, added with 0.6 g of stearic acid, mixed and tableted.

### Example 9 Sustained and controlled release capsule base on microtablets

Sustained release microtablets

| **name** | **dose** |
|---|---|
| olaparib | 8g |
| copolyvidone (PVP VA64) | 22g |
| polyoxyethylene (N10) | 5g |
| ethylcellulose (10cp) | 2g |
| magnesium stearate | 0.3g |

Immediate release microtablets

| **name** | **dose** |
|---|---|
| olaparib | 8g |
| copolyvidone (PVP VA64) | 22g |
| Crosslinked polyvidone | 3g |
| magnesium stearate | 0.3g |

Sustained-release microtablets: Olaparib and copolyvidone VA64 were sieved through a 60 mesh screen for 3 times, and added into a ball grinder to be ground to give an olaparib co-grinding mixture with an average particle diameter of less than 30 µm. The co-grinding mixture, a release rate adjusting matrix polymer polyoxyethylene and ethyl cellulose in prescription amounts were sieved through a 60 mesh screen and mixed in a three-dimensional mixer at 30 rpm for 25 min, and then added with magnesium stearate and mixed for 5 min, then pressed to be microtablets having a diameter of 4 mm.

Immediate release microtablets: Olaparib and copolyvidone VA64 were sieved through a 60 mesh screen for 3 times, and added to a ball grinder to be ground to give an olaparib co-grinding mixture with an average particle diameter of less than 30 µm. The co-grinding mixture and crosslinked polyvidone in prescription amounts were passed through a 60 mesh screen and mixed in a three-dimensional mixer at 30 rpm for 25 min, then added with magnesium stearate and mixed for 5 min, then pressed to be microtablets having a diameter of 4 mm.

Capsule filling: The sustained release microtablets prepared above were filled into capsules to prepare sustained release capsules.

The prepared immediate release microtablets and the sustained release microtablets in prescription amounts were thoroughly mixed, and then filled into capsules to prepare immediate and sustained double-release capsules. The release degree was determined in the same manner as that in Example 1 by using a buffer of pH 6.8 as the release medium. The release curve is shown in Fig. 9.

### Example 10 Sustained and controlled release capsules based on microtablets

Sustained release microtablets

| **name** | **dose** |
|---|---|
| olaparib | 8g |
| 2-hydroxypropyl-β-cyclodextrin | 30g |
| Carbomer 934 | 10g |
| sodium stearyl fumarate | 0.3g |

Immediate release microtablets

| **name** | **dose** |
|---|---|
| olaparib | 8g |
| 2-hydroxypropyl-β-cyclodextrin | 30g |
| lactose | 8g |
| croscarmellose sodium | 2g |
| sodium stearyl fumarate | 0.3g |

Sustained release microtablets: Olaparib and 2-hydroxypropyl-β-cyclodextrin were sieved through a 60 mesh screen for 3 times, added with 100 ml of water, and sheared at a high speed to give a crude suspension, which was then homogenized in a high-pressure homogenizer to an average particle diameter of less than 1000 nm, and then the nanocrystal solution was lyophilized in a lyophilizer to remove moisture. The nanocrystalline powders were passed through a 60 mesh screen and sieved with a release rate adjusting matrix polymer carbomer 934 in prescription amounts through a 60 mesh screen and mixed in a three-dimensional mixer at 30 rpm for 25 min, added with sodium stearyl fumarate and mixed for 5 min, and pressed into microtablets with a diameter of 3 mm.

Immediate release microtablets: Olaparib and 2-hydroxypropyl-β-cyclodextrin were sieved through a 60 mesh screen for 3 times, added with 100 ml of water, and sheared at a high speed to give a crude suspension, which was then homogenized in a high-pressure homogenizer to an average particle diameter of less than 1000 nm, and then the nanocrystal solution was lyophilized in a lyophilizer to remove moisture. The nanocrystalline powders were passed through a 60 mesh screen and sieved with lactose, croscarmellose sodium in prescription amounts through a 60 mesh screen and mixed in a three-dimensional mixer at 30 rpm for 25 min, and then added with sodium stearyl fumarate and mixed for 5 min, and pressed into microtablets with a diameter of 3 mm.

Capsule filling: The sustained release microtablets prepared above were filled into capsules to prepare sustained release capsules.

The prepared immediate release microtablets and the sustained release microtablets in prescription amounts were thoroughly mixed, and then filled into capsules to prepare immediate and sustained double-release capsules.

### Example 11 Single layer osmotic pump controlled release tablets

| **Components of cores** | **dose** |
|---|---|
| olaparib | 50g |
| Hypromellose E5 | 150 |
| polyvidone(K90) | 52g |
| polyvidone (K30) | 30g |
| Sodium chloride | 100g |
| sodium dodecyl sulfate | 5g |
| magnesium stearate | 4g |

Olaparib and hypromellose E5 in prescription amounts were passed through a 60 mesh screen and mixed in a three-dimensional mixer at 30 rpm for 25 min, and then slowly added into a preheated melt extruder. The extrudate was collected and pulverized through a 60 mesh screen to give an olaparib solid dispersion. The olaparib solid dispersion prepared above was sieved through a 60 mesh screen with other excipients except magnesium stearate and uniformly mixed in a three-dimensional mixer, and then mixed with magnesium stearate for 5 min to give a drug layer composition to be ready for tableting.

The cores of the single layer osmotic pump tablets were prepared by directly pressing the above-described drug layer composition. The pressed cores were coated with a 4% cellulose acetate - 0.2% PEG4000 solution to be coated with a controlled release layer with weight increment of 5% to give the single-layer osmotic pump controlled release tablets. The release degree was determined in the same manner as that in Example 1 by using a buffer of pH 6.8 as the release medium. The release curve is shown in Fig. 9.

### Comparative Example 1 Immediate release capsules

Preparation method: At 65 °C, 10 wt% of olaparib was dispersed in Glucire 44/14, stirred for 12 h, and then filled into hypromellose capsules (0#) at about 60 °C.

The dissolution degree was determined on the apparatus I of the dissolution test method (Chinese Pharmacopoeia 2010, second edition, appendix X, C). The measurements were operated following the method at 37 °C, using 500mL of Tris buffer of pH 6.8 as the release medium, at a rotary speed of 100 rpm, 6 mL of the solution was taken at 15, 30, 45, 60, 75, 90, 105, 120 min, and centrifuged, the supernatant was taken as the test solution to determine the release degree.

According to the ultraviolet-visible spectrophotometry (Chinese Pharmacopoeia 2010, second edition, appendix IV, A), the absorbance was measured at a wavelength of 278 nm, so as to measure the release degree of the capsule.

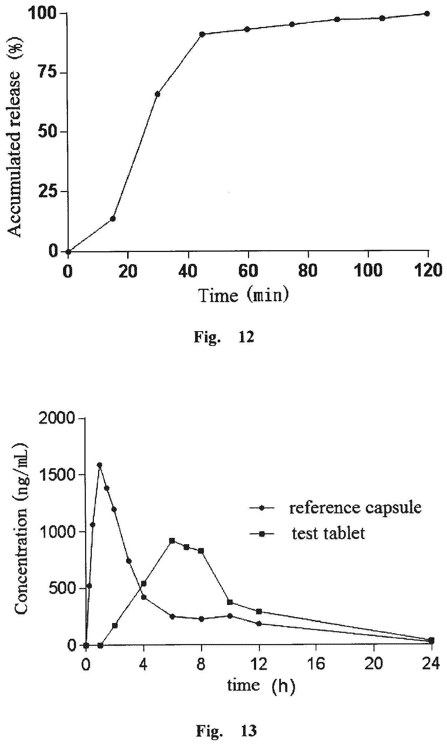

The release results were shown in Fig. 12. The active ingredient olaparib in the immediate release capsule was released more than 90% in about 45 min, and completely released within 2 h.

### Experimental Example 1

The olaparib capsule of Comparative Example 1 (reference capsule) and the double-layer osmotic pump controlled release tablet of Example 1 (test tablet) were separately administered to fed beagle dogs (n=3) with 50 mL of water. 1 mL of the extremities venous blood was taken before administration (0 h) and 0.25, 0.5, 1.0, 1.5, 2.0, 3.0, 4.0, 6.0, 8.0, 10, 12 and 24 h after administration of the capsule preparation, 1 mL of the extremities venous blood was taken before administration (0 h) and 1.0, 2.0, 4.0, 6.0, 8.0, 10, 12 and 24 h after administration of the double-layer osmotic pump controlled release tablets. The blood samples were centrifuged for 10 min at 4 °C under 4000 rpm, and then, the upper plasma was taken for the plasma concentration detection on LC-MS. The results were shown in Fig. 13.

Compared with T_{1/2} (2.6 h), Cₘₐₓ (1590.1 ng/mL) and AUCo-h (7155.7 h*ng/mL) of the capsule preparation, the double-layer osmotic pump controlled release tablets have an extended T_{1/2} of 6.1 h with a prolongation of about 135%; a decreased Cₘₐₓ of 922.9 ng/mL with a decrease of about 42%; and an AUC₀₋ₕ of 7881.3 h*ng/mL with a change of <10%.

### Experimental Example 2

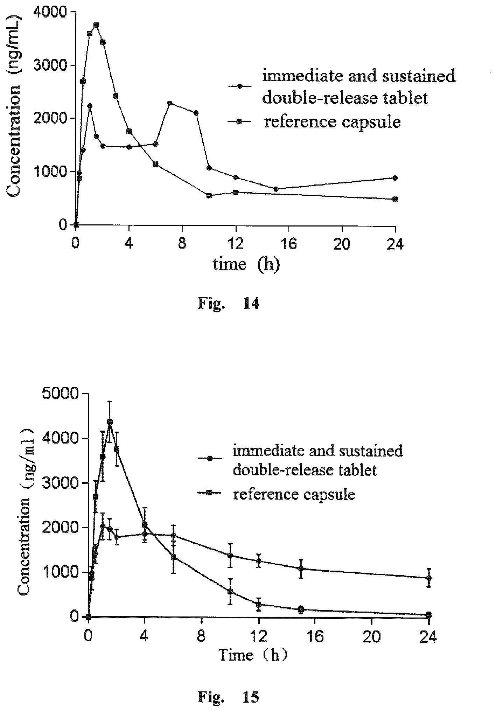

100 mg equivalent of the olaparib capsule of Comparative Example 1 (reference capsule) and the immediate and sustained double-release matrix type double-layer tablets of Example 4 (immediate and sustained double-release tablet) were respectively administered to fed beagle dogs (n =3) with 25mL of water. Blood was taken at the scheduled time after administration, and the blood samples were centrifuged at 4 °C at 4000 rpm for 10 min, then the upper plasma was taken for the plasma concentration detection on LC-MS. The results were shown in Fig. 14.

Compared with the Cₘₐₓ (4374.9 ng/mL), T_{1/2} (3.84h) and AUC₀₋ₕ (22570 h*ng/mL) of the capsule preparation, the immediate and sustained double-release matrix type double-layer tablets have a reduced Cₘₐₓ of 2397.2 ng/mL with a decrease of about 45%; an extended T_{1/2} of 9.92 h with an increase of 158%; and an AUC₀₋ₕ of 29110 h*ng/mL with a change of <30%. From the plasma concentration-time curve in Fig. 14, it can be seen that, compared with the immediate release capsule, the immediate and sustained double-release matrix type double-layer tablets can maintain a stable plasma concentration at high level for a long time, which is beneficial for performing the enzyme inhibition effect and anti-tumor effect, while providing a larger dose space for drug dose climbing and optimal drug performance.

### Experimental Example 3

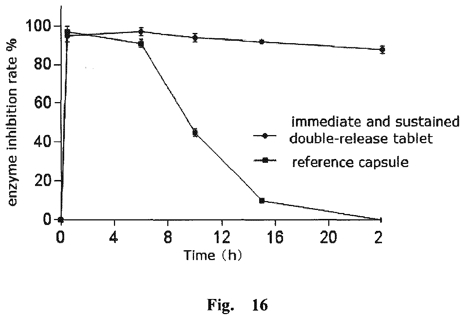

The olaparib capsule of Comparative Example 1 (reference capsule) and the immediate and sustained double-release double-layer osmotic pump tablet of Example 2 (immediate and sustained double-release preparation) were separately administered to fed beagle dogs (n=3) with 25 mL of water respectively. Blood was taken at the scheduled time after administration, and the blood samples were centrifuged at 4 °C at 4000 rpm for 10 min, then the upper plasma was taken for the plasma concentration detection on LC-MS. The results were shown in Fig. 15. PBMC were extracted from whole blood samples taken at 0h, 0.5h, 6h, 10h, 15h, and 24h, and PARP enzyme inhibition was detected by Trevigen HT PARP in vivo Pharmacodynamic Assay II kit. The results are shown in Fig. 16.

Compared with the Cₘₐₓ (4576.3 ng/mL), T1/2 (3.42h) and AUC₀₋ₕ (25163 h*ng/mL) of the capsule preparation, the immediate and sustained double-release double-layer osmotic pump tablets have a reduced Cₘₐₓ of 2154.2 ng/mL with a decrease of about 53%; an extended T_{1/2} of 12.96 h with an increase of 279%; and an AUC₀₋ₕ of 32259 h*ng/mL. The enzyme inhibition of the immediate release capsules was less than 50% at 10h, while the enzyme inhibition of the immediate and sustained double-release double-layer osmotic pump tablets was greater than 90% at 10h, and the time of the enzyme inhibition level greater than IC90 was up to 10h. From the plasma concentration-time curve in Figs. 15 and 16, it can still be seen that, compared with the immediate release capsule, the immediate and sustained double-release double-layer osmotic pump tablets can maintain a stable plasma concentration at high level for a long time, which is beneficial for performing the enzyme inhibition effect and anti-tumor effect, while providing a larger dose space for drug dose climbing and optimal drug performance.

## Claims

1. An olaparib oral sustained and controlled release pharmaceutical composition, which comprises an olaparib in an improved dissolution form; and a release rate adjusting matrix polymer,
wherein the olaparib oral sustained and controlled release pharmaceutical composition has a steady-state plasma concentration valley value C_{min,ss} of 0.2 to 4 µg/mL; and a steady-state plasma concentration peak value C_{max,ss} of 0.8 to 15 µg/mL,
wherein the olaparib in an improved dissolution form is selected from the group consisting of an olaparib salt, an olaparib co-grinding mixture, an olaparib nanocrystal and an olaparib solid dispersion; and
the release rate adjusting matrix polymer is selected from the group consisting of a cellulose derivative, a starch or a derivative thereof, an alginate, an acrylic or methacrylic acid derivative, a polyethylene oxide, a gum, and a carbohydrate-based polymer, preferably, one or a combination of two or more selected from the group consisting of hydroxypropyl cellulose, polyethylene glycol, hypromellose, methylcellulose, hydroxyethylcellulose, ethylcellulose, cellulose acetate, sodium alginate, povidone, copolyvidone, acrylic resin, carbomer, preferably one or a combination of two or more selected from the group consisting of hydroxypropylcellulose, sodium alginate, hypromellose, and carbomer;
preferably, the olaparib salt is selected from the group consisting of hydrochloride, besylate, sulfate, maleate, and camphorate;
preferably, the olaparib co-grinding mixture consists of the pharmaceutically active ingredient olaparib, a matrix polymer for solubilization and other additives, and is prepared by co-grinding the components; in the co-grinding mixture, based on the total weight of the co-grinding mixture, the olaparib is 5 to 60 wt%, preferably 20 to 40 wt%, and the matrix polymer for solubilization is 40 to 95 wt%, preferably 40 to 80 wt%, the other additives is 0 to 15 wt%, preferably 0.2 to 10 wt%;
preferably, the olaparib nanocrystal consists of the pharmaceutically active ingredient olaparib, a matrix polymer for solubilization, and/or other additives, and is obtained by prepared the components into nanosized particles by high pressure homogenization or coprecipitation; in the olaparib nanocrystal, based on the total weight of the olaparib nanocrystal, the olaparib is 10 to 99 wt%, preferably 20 to 50 wt%; the matrix polymer for solubilization is 1 to 75 wt%, preferably 1 to 65 wt%, and the other additives are 0 to 10 wt%, preferably 0 to 5 wt%; preferably the nanocrystal has a particle size of 50 to 1000 nm;
preferably, the solid dispersion consists of the pharmaceutically active ingredient olaparib, a matrix polymer for solubilization and other additives, and is prepared by solvent evaporation or melt extrusion, in the solid dispersion, based on the total weight of the solid dispersion, olaparib is 5 to 50 wt%, preferably 10 to 40 wt%, more preferably 20 to 40 wt%, the matrix polymer for solubilization is 45 to 95 wt%, preferably 50 to 80 wt%, and the other additives are 0 to 12 wt%, preferably 0 to 10 wt%;
preferably, the matrix polymer for solubilization is one or a combination of two or more selected from the group consisting of povidone, copovidone, polyoxyethylene, Soluplus®, hypromellose phthalate, hydroxypropylcellulose succinate acetate, polyethylene glycol, poloxamer, polymethacrylic acid, polyethyl acrylate, 2-hydroxypropyl-β-cyclodextrin, hypromellose, polymethacrylate, hydroxypropyl cellulose, cellulose acetate phthalate, and other pharmaceutically acceptable polymers for solubilization;
preferably, the other additives are one or a combination of two or more selected from the group consisting of pharmaceutically acceptable surfactants, a lubricant, a colloidal silica, a plasticizer, wherein the surfactant is preferably polyethylene glycol stearate or sodium lauryl sulfate.

2. An olaparib oral sustained and controlled release pharmaceutical composition according to claim 1, wherein the olaparib oral sustained and controlled release pharmaceutical composition has a steady-state plasma concentration valley value C_{min,ss} of 0.5 to 3 µg/mL; and a steady-state plasma concentration peak value C_{max,ss} of 1 to 12 µg/mL, and the steady plasma concentration peak to valley ratio is preferably less than 6, more preferably less than 4.

3. An olaparib oral sustained and controlled release pharmaceutical composition according to claim 1 or 2, wherein the olaparib pharmaceutical composition has a controlled release behavior, and the release behavior and release amount thereof in a predetermined period of time are controllable in a release medium meeting sink conditions, when the release behavior is measured in a buffer solution with a pH of 1.2 to 7.8 at 37°C using the apparatus II of the dissolution test method in the Chinese Pharmacopoeia, the release amount in 1 h is less than 50%, preferably less than 40%, more preferably 10 to 30% of the total amount of olaparib; and the release amount in 16 h is greater than 80% of the total, preferably >90% of the total amount of olaparib.

4. The olaparib oral sustained and controlled release pharmaceutical composition according to any one of claims 1 to 3, wherein the composition comprises 50 to 900 parts by weight, preferably 80 to 700 parts by weight, more preferably 120 to 600 parts by weight, of an olaparib in an improved dissolution form; and 0.1 to 300 parts by weight, preferably 20 to 250 parts by weight, more preferably 50 to 180 parts by weight, of the release rate adjusting matrix polymer;
preferably, the olaparib oral sustained and controlled release pharmaceutical composition further comprises 1 to 400 parts by weight, preferably 2 to 300 parts by weight, more preferably 5 to 250 parts by weight of other additives;
preferably, the olaparib oral sustained and controlled release pharmaceutical composition comprises:
50 to 600 parts by weight of an olaparib salt, and 10 to 250 parts by weight of a release rate adjusting matrix polymer, preferably further comprising 1 to 300 parts by weight of other additives; or
50 to 700 parts by weight of an olaparib co-grinding mixture, and 10 to 200 parts by weight of a release rate adjusting matrix polymer, preferably further comprising 1 to 150 parts by weight of other additives; or
50 to 800 parts by weight of an olaparib nanocrystal, and 0.1 to 250 parts by weight of a release rate adjusting matrix polyme, preferably further comprising 1 to 200 parts by weight of other additives r; or
50 to 900 parts by weight of an olaparib solid dispersion, and 20 to 300 parts by weight of a release rate adjusting matrix polymer, preferably further comprising 1 to 200 parts by weight of other additives,
wherein, the other additives are pharmaceutical excipients, preferably selected from the group consisting of release regulators, semi-permeable controlled release coating materials, seal coating materials, solubilizing agents, disintegrants, coating powders, plasticizers, porogens, expansive materials, fillers, osmotic pressure regulators, lubricants, adhesives, dyes, anti-adherents, opacifiers, diluents and/or other pharmaceutically acceptable additives.

5. The olaparib oral sustained and controlled release pharmaceutical composition according to any one of claims 1 to 4, which is a sustained and controlled release preparation containing a single sustained release phase or an immediate and sustained double release preparation containing both immediate release phase and sustained release phase, wherein,
preferably, the sustained release phase is selected from the group consisting of a controlled release tablet, a controlled release pellet, a controlled release composition in a tablet, a controlled release composition in a tablet or pellet core, a controlled release layer composition incorporated into a double layer tablet, and any combination thereof;
preferably, the immediate release phase is selected from the group consisting of an immediate release tablet, an immediate release pellet, an immediate release composition in a tablet, an immediate release coat layer wrapping around a controlled release tablet or pellet core, and an immediate release layer composition in a double-layer controlled release tablet and any combination thereof.

6. An olaparib oral sustained and controlled release pharmaceutical composition according to claim 5, wherein in the immediate and sustained double release preparation, the pharmaceutically active ingredient in the immediate release phase accounts for 10 to 50 wt%, preferably 20 to 40 wt% of the total amount of the pharmaceutically active ingredient; the pharmaceutically active ingredient in the sustained release phase accounts for 50 to 90 wt%, preferably 60 to 80 wt% of the total amount of the pharmaceutically active ingredient.

7. An olaparib oral sustained and controlled release pharmaceutical composition according to claim 5, which is a tablet or a capsule, and is preferably selected from the group consisting of an osmotic pump controlled release tablet, an osmotic pump immediate and sustained double release tablet, a matrix type sustained release tablet, a matrix type immediate and sustained double release double layer tablet, a matrix type immediate and sustained double release coated tablet, a sustained release tablet based on sustained release pellets, an immediate and sustained double release tablet based on sustained release pellets and immediate release pellets, a capsule containing matrix type sustained release pellets, a capsule containing sustained release coated pellets, a capsule containing sustained release pellets with immediate release coat, an immediate and sustained double release capsule containing immediate release pellets and matrix type sustained release pellets, an immediate and sustained double release capsule containing immediate release pellets and sustained release coated pellets, a capsule containing matrix-type sustained release microtablets, a capsule containing matrix-type sustained release microtablets with immediate release coat, and a capsule containing immediate release microtablets and matrix type sustained release microtablets.

8. The olaparib oral sustained and controlled release pharmaceutical composition according to any one of claims 1 to 7 for use in prevention or treatment of a tumor, preferably, the tumor is selected from the group consisting of the tumors with defects in DNA repair function, preferably the tumor is selected from the group consisting of a combination of two or more cancers related to BRCA gene mutation, preferably ovarian cancer, gastric cancer and breast cancer, and a tumor associated with BRCA1 and BRCA2 gene mutation.

9. The olaparib oral sustained and controlled release pharmaceutical composition for use according to claim 8, wherein the expected total dose of the olaparib oral sustained and controlled release pharmaceutical composition which needs to be administered daily, is 100 to 1400 mg calculated by olaparib, and the amount of the pharmaceutically active ingredient olaparib contained in a single finished tablet or capsule is 20 to 400 mg, preferably 50 to 300 mg.

## Patentansprüche

1. Orale pharmazeutische Zusammensetzung mit verzögerter und kontrollierter Freisetzung von Olaparib, umfassend ein Olaparib in einer verbesserten Auflösungsform und ein die Freisetzungsrate einstellendes Matrixpolymer,
wobei die orale pharmazeutische Zusammensetzung mit verzögerter und kontrollierter Freisetzung von Olaparib einen Steady-State-Plasmakonzentrations-Talwert C_{min,ss} von 0,2 bis 4 µg/ml und einen Steady-State-Plasmakonzentrations-Spitzenwert C_{max,ss} von 0,8 bis 15 µg/ml aufweist,
wobei das Olaparib in einer verbesserten Auflösungsform ausgewählt ist aus der Gruppe bestehend aus einem Olaparib-Salz, einer Olaparib-Co-Mahlmischung, einem Olaparib-Nanokristall und einer Olaparib-Feststoffdispersion; und
das die Freisetzungsrate einstellende Matrixpolymer ausgewählt ist aus der Gruppe bestehend aus einem Cellulosederivat, einer Stärke oder einem Derivat davon, einem Alginat, einem Acryl- oder Methacrylsäurederivat, einem Polyethylenoxid, einem Gummi und einem Polymer auf Kohlenhydratbasis, vorzugsweise einem oder einer Kombination von zwei oder mehr, ausgewählt aus der Gruppe bestehend aus Hydroxypropylcellulose, Polyethylenglycol, Hypromellose, Methylcellulose, Hydroxyethylcellulose, Ethylcellulose, Celluloseacetat, Natriumalginat, Povidon, Copolyvidon, Acrylharz, Carbomer, vorzugsweise eines oder eine Kombination von zwei oder mehr, ausgewählt aus der Gruppe bestehend aus Hydroxypropylcellulose, Natriumalginat, Hypromellose und Carbomer;
vorzugsweise das Olaparib-Salz ausgewählt ist aus der Gruppe bestehend aus Hydrochlorid, Besylat, Sulfat, Maleat und Camphorat;
vorzugsweise das Olaparib-Co-Mahlgutgemisch aus dem pharmazeutischen Wirkstoff Olaparib, einem Matrixpolymer zur Solubilisierung und anderen Additiven besteht, und durch Co-Mahlen der Komponenten hergestellt wird; wobei in dem Co-Mahlgutgemisch, bezogen auf das Gesamtgewicht des Co-Mahlgutgemisches, das Olaparib 5 bis 60 Gew.-%, vorzugsweise 20 bis 40 Gew.-%, beträgt, und das Matrixpolymer zur Solubilisierung 40 bis 95 Gew.-%, vorzugsweise 40 bis 80 Gew.-%, beträgt, die anderen Additive 0 bis 15 Gew.-%, vorzugsweise 0,2 bis 10 Gew.-%, betragen;
vorzugsweise der Olaparib-Nanokristall aus dem pharmazeutischen Wirkstoff Olaparib, einem Matrixpolymer zur Solubilisierung und/oder anderen Additiven besteht und erhalten wird, indem die Komponenten durch Hochdruckhomogenisierung oder Co-Ausfällung zu nanoskaligen Partikeln aufbereitet werden; wobei in dem Olaparib-Nanokristall, bezogen auf das Gesamtgewicht des Olaparib-Nanokristalls, das Olaparib 10 bis 99 Gew.-%, vorzugsweise 20 bis 50 Gew.-%, beträgt; das Matrixpolymer zur Solubilisierung 1 bis 75 Gew.-%, vorzugsweise 1 bis 65 Gew.-%, beträgt; und die anderen Additive 0 bis 10 Gew.-%, vorzugsweise 0 bis 5 Gew.-%, betragen; vorzugsweise der Nanokristall eine Teilchengröße von 50 bis 1000 nm aufweist;
vorzugsweise die Feststoffdispersion aus dem pharmazeutischen Wirkstoff Olaparib, einem Matrixpolymer zur Solubilisierung und weiteren Additiven besteht und durch Lösungsmittelverdampfung oder Schmelzextrusion hergestellt wird, wobei in der Feststoffdispersion das Olaparib, bezogen auf das Gesamtgewicht der Feststoffdispersion, 5 bis 50 Gew.-%, vorzugsweise 10 bis 40 Gew.-%, besonders bevorzugt 20 bis 40 Gew.-%, beträgt, das Matrixpolymer zur Solubilisierung 45 bis 95 Gew.-%, vorzugsweise 50 bis 80 Gew.-%, beträgt, und die weiteren Additive 0 bis 12 Gew.-%, vorzugsweise 0 bis 10 Gew.-%, betragen;
vorzugsweise das Matrixpolymer zur Solubilisierung eines oder eine Kombination von zwei oder mehr ist, ausgewählt aus der Gruppe bestehend aus Povidon, Copovidon, Polyoxyethylen, Soluplus®, Hypromellosephthalat, Hydroxypropylcellulosesuccinatacetat Polyethylenglycol, Poloxamer, Polymethacrylsäure, Polyethylacrylat, 2-Hydroxypropyl-β-cyclodextrin, Hypromellose, Polymethacrylat, Hydroxypropylcellulose, Celluloseacetatphthalat und andere pharmazeutisch verträgliche Polymere zur Solubilisierung;
vorzugsweise die anderen Additive eines oder eine Kombination von zwei oder mehr sind, ausgewählt aus der Gruppe bestehend aus pharmazeutisch verträglichen Tensiden, einem Schmiermittel, einem kolloidalen Siliciumdioxid, einem Weichmacher, wobei das Tensid vorzugsweise Polyethylenglycolstearat oder Natriumlaurylsulfat ist.

2. Orale pharmazeutische Zusammensetzung mit verzögerter und kontrollierter Freisetzung von Olaparib nach Anspruch 1, wobei die orale pharmazeutische Zusammensetzung mit verzögerter und kontrollierter Freisetzung von Olaparib einen Steady-State-Plasmakonzentrations-Talwert C_{min,ss} von 0,5 bis 3 µg/ml und einen Steady-State-Plasmakonzentrations-Spitzenwert C_{max,ss} von 1 bis 12 µg/ml aufweist und das Steady-State-Plasmakonzentrations-Spitzenwert-Talwert-Verhältnis vorzugsweise weniger als 6, besonders bevorzugt weniger als 4 beträgt.

3. Orale pharmazeutische Zusammensetzung mit verzögerter und kontrollierter Freisetzung von Olaparib nach Anspruch 1 oder 2, wobei die pharmazeutische Zusammensetzung von Olaparib ein kontrolliertes Freisetzungsverhalten aufweist und das Freisetzungsverhalten und die Freisetzungsmenge davon in einem vorbestimmten Zeitraum in einem Freisetzungsmedium, das die Sinkbedingungen erfüllt, kontrollierbar sind, wenn das Freisetzungsverhalten in einer Pufferlösung mit einem pH-Wert von 1.2 bis 7,8 bei 37 °C unter Verwendung der Vorrichtung II der Auflösungstestmethode in der Chinese Pharmacopeia gemessen wird, ist die Freisetzungsmenge in 1 h weniger als 50%, vorzugsweise weniger als 40%, besonders bevorzugt 10 bis 30% der Gesamtmenge an Olaparib; und die Freisetzungsmenge in 16 h ist größer als 80% der Gesamtmenge, vorzugsweise >90% der Gesamtmenge an Olaparib.

4. Orale pharmazeutische Zusammensetzung mit verzögerter und kontrollierter Freisetzung von Olaparib nach einem der Ansprüche 1 bis 3, wobei die Zusammensetzung 50 bis 900 Gewichtsteile, vorzugsweise 80 bis 700 Gewichtsteile, besonders bevorzugt 120 bis 600 Gewichtsteile, eines Olaparibs in einer verbesserten Auflösungsform und 0,1 bis 300 Gewichtsteile, vorzugsweise 20 bis 250 Gewichtsteile, besonders bevorzugt 50 bis 180 Gewichtsteile, des die Freisetzungsrate einstellenden Matrixpolymers umfasst;
vorzugsweise die orale pharmazeutische Zusammensetzung mit verzögerter und kontrollierter Freisetzung von Olaparib weiterhin 1 bis 400 Gewichtsteile, vorzugsweise 2 bis 300 Gewichtsteile, besonders bevorzugt 5 bis 250 Gewichtsteile, anderer Additive umfasst;
vorzugsweise umfasst die orale pharmazeutische Zusammensetzung mit verzögerter und kontrollierter Freisetzung von Olaparib:
50 bis 600 Gewichtsteile eines Olaparib-Salzes und 10 bis 250 Gewichtsteile eines die Freisetzungsrate einstellenden Matrixpolymers, vorzugsweise ferner 1 bis 300 Gewichtsteile anderer Zusatzstoffe; oder
50 bis 700 Gewichtsteile einer Olaparib-Co-Mahlgutmischung und 10 bis 200 Gewichtsteile eines die Freisetzungsrate einstellenden Matrixpolymers, vorzugsweise weiterhin umfassend 1 bis 150 Gewichtsteile anderer Additive; oder
50 bis 800 Gew.-Teile eines Olaparib-Nanokristalls und 0,1 bis 250 Gew.-Teile eines die Freisetzungsrate einstellenden Matrixpolymers, vorzugsweise weiterhin umfassend 1 bis 200 Gew.-Teile anderer Additive; oder
50 bis 900 Gew.-Teile einer festen Olaparib-Dispersion und 20 bis 300 Gew.-Teile eines die Freisetzungsrate einstellenden Matrixpolymers, vorzugsweise weiterhin umfassend 1 bis 200 Gew.-Teile anderer Zusatzstoffe,
wobei die anderen Additive pharmazeutische Hilfsstoffe sind, vorzugsweise ausgewählt aus der Gruppe bestehend aus Freisetzungsregulatoren, semipermeablen Beschichtungsmaterialien mit kontrollierter Freisetzung, Versiegelungsbeschichtungsmaterialien, Solubilisierungsmitteln, Zerfallsmitteln, Beschichtungspulvem, Weichmachern, Porogenen, expansiven Materialien, Füllstoffen, osmotischen Druckregulatoren, Schmiermitteln, Klebstoffen, Farbstoffen, Antihaftmitteln, Trübungsmitteln, Verdünnungsmitteln und/oder anderen pharmazeutisch verträglichen Additiven.

5. Orale pharmazeutische Zusammensetzung mit verzögerter und kontrollierter Freisetzung von Olaparib nach einem der Ansprüche 1 bis 4, die eine Zubereitung mit verzögerter und kontrollierter Freisetzung ist, enthaltend eine einzelne Phase mit verzögerter Freisetzung oder eine Zubreitung mit sofortiger und verzögerter doppelter Freisetzung, enthaltend sowohl eine Phase mit sofortiger Freisetzung als auch eine Phase mit verzögerter Freisetzung, wobei
vorzugsweise die Phase mit verzögerter Freisetzung ausgewählt ist aus der Gruppe bestehend aus einer Tablette mit kontrollierter Freisetzung, einem Pellet mit kontrollierter Freisetzung, einer Zusammensetzung mit kontrollierter Freisetzung in einer Tablette, einer Zusammensetzung mit kontrollierter Freisetzung in einem Tabletten- oder Pelletkern, einer Schichtzusammensetzung mit kontrollierter Freisetzung, die in eine Doppelschichttablette eingearbeitet ist, und jeder Kombination davon;
vorzugsweise ist die Phase mit sofortiger Freisetzung ausgewählt aus der Gruppe bestehend aus einer Tablette mit sofortiger Freisetzung, einem Pellet mit sofortiger Freisetzung, einer Zusammensetzung mit sofortiger Freisetzung in einer Tablette, einer Mantelschicht mit sofortiger Freisetzung, die einen Tabletten- oder Pelletkern mit kontrollierter Freisetzung umhüllt, und einer Schichtzusammensetzung mit sofortiger Freisetzung in einer zweischichtigen Tablette mit kontrollierter Freisetzung und jeder Kombination davon.

6. Orale pharmazeutische Zusammensetzung mit verzögerter und kontrollierter Freisetzung von Olaparib nach Anspruch 5, wobei in der Zubereitung mit sofortiger und verzögerter doppelter Freisetzung der pharmazeutische Wirkstoff in der Phase der sofortigen Freisetzung 10 bis 50 Gew.-%, vorzugsweise 20 bis 40 Gew.-%, der Gesamtmenge des pharmazeutischen Wirkstoffs ausmacht; der pharmazeutische Wirkstoff in der Phase der verzögerten Freisetzung 50 bis 90 Gew.-%, vorzugsweise 60 bis 80 Gew.-%, der Gesamtmenge des pharmazeutischen Wirkstoffs ausmacht.

7. Orale pharmazeutische Zusammensetzung mit verzögerter und kontrollierter Freisetzung von Olaparib nach Anspruch 5, die eine Tablette oder eine Kapsel ist und vorzugsweise ausgewählt ist aus der Gruppe bestehend aus einer Tablette mit kontrollierter Freisetzung mit osmotischer Pumpe, einer Tablette mit sofortiger und verzögerter doppelter Freisetzung mit osmotischer Pumpe, einer Tablette mit verzögerter Freisetzung vom Matrixtyp, einer Doppelschichttablette mit sofortiger und verzögerter doppelter Freisetzung vom Matrixtyp, einer überzogenen Tablette mit sofortiger und verzögerter doppelter Freisetzung vom Matrixtyp, einer Tablette mit verzögerter Freisetzung auf Basis von Pellets mit verzögerter Freisetzung, einer Tablette mit sofortiger und verzögerter doppelter Freisetzung auf Basis von Pellets mit verzögerter Freisetzung und Pellets mit sofortiger Freisetzung, eine Kapsel, enthaltend Pellets mit verzögerter Freisetzung vom Matrixtyp, eine Kapsel, enthaltend beschichtete Pellets mit verzögerter Freisetzung, eine Kapsel, enthaltend Pellets mit verzögerter Freisetzung mit einem Überzug mit sofortiger Freisetzung, eine Kapsel mit sofortiger und verzögerter doppelter Freisetzung, enthaltend Pellets mit sofortiger Freisetzung und Pellets mit verzögerter Freisetzung vom Matrixtyp, eine Kapsel mit sofortiger und verzögerter doppelter Freisetzung, enthaltend Pellets mit sofortiger Freisetzung und beschichtete Pellets mit verzögerter Freisetzung, eine Kapsel, enthaltend Mikrotabletten mit verzögerter Freisetzung vom Matrixtyp, eine Kapsel, enthaltend Mikrotabletten mit verzögerter Freisetzung vom Matrixtyp mit einem Überzug mit sofortiger Freisetzung, und eine Kapsel, enthaltend Mikrotabletten mit sofortiger Freisetzung und Mikrotabletten mit verzögerter Freisetzung vom Matrixtyp.

8. Orale pharmazeutische Zusammensetzung mit verzögerter und kontrollierter Freisetzung von Olaparib nach einem der Ansprüche 1 bis 7 zur Verwendung bei der Vorbeugung oder Behandlung eines Tumors, wobei der Tumor vorzugsweise aus der Gruppe ausgewählt ist bestehend aus Tumoren mit Defekten in der DNA-Reparaturfunktion, wobei der Tumor vorzugsweise aus der Gruppe ausgewählt ist bestehend aus einer Kombination von zwei oder mehr Krebsarten, die mit einer BRCA-Genmutation verbunden sind, vorzugsweise Eierstockkrebs, Magenkrebs und Brustkrebs, und einem Tumor, der mit einer BRCA1- und BRCA2-Genmutation verbunden ist.

9. Orale pharmazeutische Zusammensetzung mit verzögerter und kontrollierter Freisetzung von Olaparib zur Verwendung nach Anspruch 8, wobei die erwartete Gesamtdosis der oralen pharmazeutischen Zusammensetzung mit verzögerter und kontrollierter Freisetzung von Olaparib, die täglich verabreicht werden muss, 100 bis 1400 mg, berechnet nach Olaparib, beträgt und die Menge des pharmazeutischen Wirkstoffs Olaparib, die in einer einzelnen fertigen Tablette oder Kapsel enthalten ist, 20 bis 400 mg, vorzugsweise 50 bis 300 mg, beträgt.

## Revendications

1. Composition pharmaceutique à libération prolongée et contrôlée par voie orale d'olaparib, qui comprend un olaparib sous une forme à dissolution améliorée ; et un polymère de matrice d'ajustement de la vitesse de libération,
la composition pharmaceutique à libération prolongée et contrôlée par voie orale d'olaparib ayant une valeur de vallée de concentration plasmatique à l'état stable C_{min,ss} de 0,2 à 4 µg/ml ; et une valeur pic de concentration plasmatique à l'état stable C_{max,ss} de 0,8 à 15 µ/ml,
l'olaparib sous une forme à dissolution améliorée étant sélectionné dans le groupe constitué d'un sel d'olaparib, d'un mélange de co-broyage d'olaparib, d'un nanocristal d'olaparib et d'une dispersion solide d'olaparib ; et
le polymère de matrice d'ajustement de la vitesse de libération étant sélectionné dans le groupe constitué d'un dérivé de cellulose, d'un amidon ou d'un dérivé de celui-ci, d'un alginate, d'un dérivé d'acide acrylique ou méthacrylique, d'un poly(oxyde d'éthylène), d'une gomme, et d'un polymère à base d'hydrate de carbone, préférablement, d'un ou d'une combinaison de deux ou plusieurs sélectionnés dans le groupe constitué de l'hydroxypropyl cellulose, du poly(éthylène glycol), de l'hypromellose, de la méthylcellulose, de l'hydroxyéthylcellulose, de l'éthylcellulose, de l'acétate de cellulose, de l'alginate de sodium, de la povidone, de la copolyvidone, de la résine acrylique, d'un carbomère, préférablement de l'un ou d'une combinaison de deux ou plusieurs sélectionnés dans le groupe constitué de l'hydroxypropylcellulose, de l'alginate de sodium, de l'hypromellose, et d'un carbomère ;
préférablement, le sel d'olaparib étant sélectionné dans le groupe constitué du chlorhydrate, bésylate, sulfate, maléate, et camphorate ;
préférablement, le mélange de co-broyage d'olaparib étant constitué de l'ingrédient pharmaceutiquement actif olaparib, d'un polymère de matrice pour la solubilisation et d'autres additifs, et est préparé par co-broyage des composants ; dans le mélange de co-broyage, sur la base du poids total du mélange de co-broyage, l'olaparib étant de 5 à 60 % en pds, préférablement de 20 à 40 % en pds, et le polymère de matrice pour la solubilisation étant de 40 à 95 % en pds, préférablement de 40 à 80 % en pds, les autres additifs étant de 0 à 15 % en pds, préférablement de 0,2 à 10 % en pds ;
préférablement, le nanocristal d'olaparib est constitué de l'ingrédient pharmaceutiquement actif olaparib, d'un polymère de matrice pour la solubilisation, et/ou d'autres additifs, et est obtenu en préparant les composants en particules à taille de l'ordre du nanomètre par homogénéisation à haute pression ou coprécipitation ; en nanocristal d'olaparib, sur la base du poids total du nanocristal d'olaparib, l'olaparib étant de 10 à 99 % en pds, préférablement de 20 à 50 % en pds ; le polymère de matrice pour la solubilisation étant de 1 à 75 % en pds, préférablement de 1 à 65 % en pds, et les autres additifs étant de 0 à 10 % en pds, préférablement de 0 à 5 % en pds ; préférablement le nanocristal ayant une taille de particule de 50 à 1 000 nm ;
préférablement, la dispersion solide est constituée de l'ingrédient pharmaceutiquement actif olaparib, d'un polymère de matrice pour la solubilisation et d'autres additifs, et est préparée par évaporation de solvant ou extrusion à l'état fondu, dans la dispersion solide, sur la base du poids total de la dispersion solide, l'olaparib étant de 5 à 50 % en pds, préférablement de 10 à 40 % en pds, plus préférablement de 20 à 40 % en pds, le polymère de matrice pour la solubilisation étant de 45 à 95 % en pds, préférablement de 50 à 80 % en pds, et les autres additifs étant de 0 à 12 % en pds, préférablement de 0 à 10 % en pds ;
préférablement, le polymère de matrice pour la solubilisation est l'un ou une combinaison de deux ou plusieurs sélectionnés dans le groupe constitué de la povidone, de la copovidone, du polyoxyéthylène, du Soluplus®, du phtalate d'hypromellose, du succinate acétate d'hydroxypropylcellulose, du poly(éthylène glycol), du poloxamère, du poly(acide méthacrylique), du poly(acrylate d'éthyle), de la 2-hydroxypropyl-β-cyclodextrine, de l'hypromellose, du polyméthacrylate, de l'hydroxypropyl cellulose, de l'acétate phtalate de cellulose, et d'autres polymères pharmaceutiquement acceptables pour la solubilisation ;
préférablement, les autres additifs sont l'un ou une combinaison de deux ou plusieurs sélectionnés dans le groupe constitué des tensioactifs pharmaceutiquement acceptables, d'un lubrifiant, d'une silice colloïdale, d'un agent plastifiant, le tensioactif étant préférablement le stéarate de poly(éthylène glycol) ou le lauryl sulfate de sodium.

2. Composition pharmaceutique à libération prolongée et contrôlée par voie orale d'olaparib selon la revendication 1, la composition pharmaceutique à libération prolongée et contrôlée par voie orale d'olaparib ayant une valeur de vallée de concentration plasmatique à l'état stable C_{min,ss} de 0,5 à 3 µg/ml ; et une valeur pic de concentration plasmatique à l'état stable C_{max,ss} de 1 à 12 µg/ml, et le rapport pic à vallée des concentrations plasmatiques stables étant préférablement inférieur à 6, plus préférablement inférieur à 4.

3. Composition pharmaceutique à libération prolongée et contrôlée par voie orale d'olaparib selon la revendication 1 ou 2, la composition pharmaceutique d'olaparib ayant un comportement de libération contrôlée, et le comportement de libération et sa quantité de libération dans une période prédéterminée de temps pouvant être contrôlés dans un milieu de libération satisfaisant les conditions Sink, le comportement de libération étant mesuré dans une solution tampon avec un pH de 1,2 à 7,8 à 37°C en utilisant l'appareil II du procédé de test de dissolution dans la Chinese Pharmacopoeia, la quantité de libération en 1 h étant inférieure à 50 %, préférablement inférieure à 40 %, plus préférablement de 10 à 30 % de la quantité totale d'olaparib ; et la quantité de libération en 16 h étant supérieure à 80 % du total, préférablement > 90 % de la quantité totale d'olaparib.

4. Composition pharmaceutique à libération prolongée et contrôlée par voie orale d'olaparib selon l'une quelconque des revendications 1 à 3, la composition comprenant de 50 à 900 parties en poids, préférablement de 80 à 700 parties en poids, plus préférablement de 120 à 600 parties en poids, d'un olaparib sous une forme de dissolution améliorée ; et de 0,1 à 300 parties en poids, préférablement de 20 à 250 parties en poids, plus préférablement de 50 à 180 parties en poids, du polymère de matrice d'ajustement de la vitesse de libération ;
préférablement, la composition pharmaceutique à libération prolongée et contrôlée par voie orale d'olaparib comprenant en outre de 1 à 400 parties en poids, préférablement de 2 à 300 parties en poids, plus préférablement de 5 à 250 parties en poids d'autres additifs ;
préférablement, la composition pharmaceutique à libération prolongée et contrôlée par voie orale d'olaparib comprenant :
de 50 à 600 parties en poids d'un sel d'olaparib, et de 10 à 250 parties en poids d'un polymère de matrice d'ajustement de la vitesse de libération, comprenant en outre préférablement de 1 à 300 parties en poids d'autres additifs ; ou
de 50 à 700 parties en poids d'un mélange de co-broyage d'olaparib, et de 10 à 200 parties en poids d'un polymère de matrice d'ajustement de la vitesse de libération, comprenant en outre préférablement de 1 à 150 parties en poids d'autres additifs ; ou
de 50 à 800 parties en poids d'un nanocristal d'olaparib, et de 0,1 à 250 parties en poids d'un polymère de matrice d'ajustement de la vitesse de libération, comprenant en outre préférablement de 1 à 200 parties en poids d'autres additifs ; ou
de 50 à 900 parties en poids d'une dispersion solide d'olaparib, et de 20 à 300 parties en poids d'un polymère de matrice d'ajustement de la vitesse de libération, comprenant en outre préférablement de 1 à 200 parties en poids d'autres additifs,
les autres additifs étant des excipients pharmaceutiques, préférablement sélectionnés dans le groupe constitué de régulateurs de libération, de matériaux d'enrobage semi-perméable à libération contrôlée, de matériaux d'enrobage d'étanchéité, d'agents de solubilisation, d'agents délitants, de poudres d'enrobage, d'agents plastifiants, d'agents porogènes, de matériaux d'expansion, de charges, d'agents de régulation de la pression osmotique, d'agents lubrifiants, d'adhésifs, de colorants, d'agents anti-adhérents, d'agents opacifiants, de diluants et/ou d'autres additifs pharmaceutiquement acceptables.

5. Composition pharmaceutique à libération prolongée et contrôlée par voie orale d'olaparib selon l'une quelconque des revendications 1 à 4, qui est une préparation à libération prolongée et contrôlée contenant une préparation à phase unique à libération prolongée ou à double libération immédiate et prolongée contenant à la fois la phase à libération immédiate et la phase à libération prolongée,
préférablement, la phase à libération prolongée étant sélectionnée dans le groupe constitué d'un comprimé à libération contrôlée, d'une pastille à libération contrôlée, d'une composition à libération contrôlée dans un comprimé, d'une composition à libération contrôlée dans un cœur de comprimé ou de pastille, d'une composition à couche de libération contrôlée incorporée dans un comprimé double couche, et de n'importe quelle combinaison de ceux-ci ;
préférablement, la phase à libération immédiate étant sélectionnée dans le groupe constitué d'un comprimé à libération immédiate, d'une pastille à libération immédiate, d'une composition à libération immédiate dans un comprimé, d'une couche d'enrobage à libération immédiate enveloppée autour d'un cœur de comprimé ou de pastille à libération contrôlée, et d'une composition de couche à libération immédiate dans un comprimé double couche à libération contrôlée et de n'importe quelle combinaison de ceux-ci.

6. Composition pharmaceutique à libération prolongée et contrôlée par voie orale d'olaparib selon la revendication 5, dans la préparation à double libération immédiate et prolongée, l'ingrédient pharmaceutiquement actif dans la phase à libération immédiate comptant pour 10 à 50 % en pds, préférablement de 20 à 40 % en pds de la quantité totale de l'ingrédient pharmaceutiquement actif ; l'ingrédient pharmaceutiquement actif dans la phase à libération prolongée comptant pour 50 à 90 % en pds, préférablement de 60 à 80 % en pds de la quantité totale de l'ingrédient pharmaceutiquement actif.

7. Composition pharmaceutique à libération prolongée et contrôlée par voie orale d'olaparib selon la revendication 5, qui est un comprimé ou une capsule, et qui est préférablement sélectionnée dans le groupe constitué d'un comprimé à libération contrôlée à pompe osmotique, d'un comprimé à double libération immédiate et prolongée à pompe osmotique, d'un comprimé de type matrice à libération prolongée, d'un comprimé à double couche de type matrice à double libération immédiate et prolongée, d'un comprimé enrobé de type matrice à double libération immédiate et prolongée, d'un comprimé à libération prolongée à base de pastilles à libération prolongée, d'un comprimé à double libération immédiate et prolongée à base de pastilles à libération prolongée et de pastilles à libération immédiate, d'une capsule contenant des pastilles à libération prolongée de type matrice, d'une capsule contenant des pastilles enrobées à libération prolongée, d'une capsule contenant des pastilles à libération prolongée ayant un enrobage à libération immédiate, d'une capsule à double libération immédiate et prolongée contenant des pastilles à libération immédiate et des pastilles de type matrice à libération prolongée, d'une capsule à double libération immédiate et prolongée contenant des pastilles à libération immédiate et des pastilles enrobées à libération prolongée, d'une capsule contenant des microcomprimés de type matrice à libération prolongée, d'une capsule contenant des microcomprimés de type matrice à libération prolongée ayant un enrobage à libération immédiate, et d'une capsule contenant des microcomprimés à libération immédiate et des microcomprimés de type matrice à libération prolongée.

8. Composition pharmaceutique à libération prolongée et contrôlée par voie orale d'olaparib selon l'une quelconque des revendications 1 à 7 pour l'utilisation dans la prévention ou le traitement d'une tumeur, préférablement, la tumeur étant sélectionnée dans le groupe constitué des tumeurs relative à des défauts dans la fonction de réparation de l'ADN, préférablement la tumeur étant sélectionnée dans le groupe constitué d'une combinaison de deux ou plusieurs cancers liés à la mutation du gène BRCA, préférablement le cancer de l'ovaire, le cancer de l'estomac et le cancer du sein, et une tumeur associée à la mutation du gène BRCA1 et BRCA2.

9. Composition pharmaceutique à libération prolongée et contrôlée par voie orale d'olaparib pour l'utilisation selon la revendication 8, la dose totale prévue de la composition pharmaceutique à libération prolongée et contrôlée par voie orale d'olaparib qui a besoin d'être administrée quotidiennement, étant de 100 à 1 400 mg calculée par olaparib, et la quantité de l'ingrédient pharmaceutiquement actif olaparib contenu dans un comprimé ou une capsule fini·e unique étant de 20 à 400 mg, préférablement de 50 à 300 mg.
